(19) Europäisches Patentamt
European Patent Office
Office européen des brevets

(11) Veröffentlichungsnummer: **0 237 695**
**A2**

(12) **EUROPÄISCHE PATENTANMELDUNG**

(21) Anmeldenummer: **87100070.9**

(22) Anmeldetag: **06.01.87**

(51) Int. Cl.⁴: **C07D 417/10** , C07D 417/14 , C07D 491/04 , C07D 491/14 , A61K 31/44

(30) Priorität: **17.01.86 DE 3601196**

(43) Veröffentlichungstag der Anmeldung: **23.09.87 Patentblatt 87/39**

(84) Benannte Vertragsstaaten: **AT BE CH DE ES FR GB IT LI NL SE**

(71) Anmelder: **Merck Patent Gesellschaft mit beschränkter Haftung**
**Frankfurter Strasse 250**
**D-6100 Darmstadt(DE)**

(72) Erfinder: **Juraszyk, Horst, Dr.**
**Kleiner Ring 14**
**D-6104 Seeheim 1(DE)**
Erfinder: **Gericke, Rolf, Dr.**
**Mozartstrasse 19**
**D-6104 Seeheim 1(DE)**
Erfinder: **Lues, Inge, Dr.**
**Paul Wagner Strasse 13**
**D-6100 Darmstadt(DE)**
Erfinder: **Bergmann, Rolf, Dr.**
**Birkenhag 36**
**D-6101 Reichelsheim(DE)**
Erfinder: **Schmitges, Claus J., Dr.**
**Karolinger Strasse 5**
**D-6114 Gross-Umstadt(DE)**

(54) **1,4-Dihydropyridine.**

(57) Neue 1,4-Dihydropyridine der Formel I

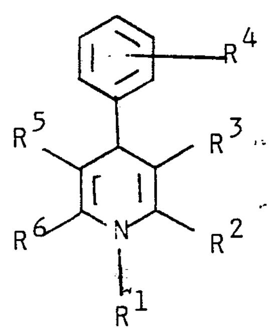

I

worin

$R^1$ bis $R^6$ die in Patentanspruch 1 angegebene Bedeutung haben, sowie deren Salze beeinflussen den Calcium-Influx in die Zelle.

EP 0 237 695 A2

## 1,4-Dihydropyridene

Die Erfindung betrifft neue 1,4-Dihydropyridine der allgemeinen Formel I,

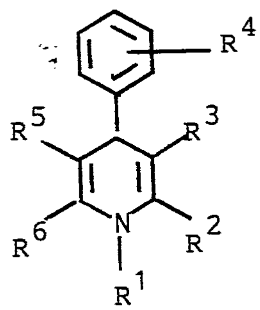

I

worin

$R^1$ H, A, Ar-alkyl, AO-alkyl, ArO-alkyl, Ar-alkyl-O-alkyl oder $R^7R^8$N-alkyl,

$R^2$ und $R^6$ jeweils H, A, Ar-alkyl, Hal-alkyl, $CF_3$, $R^9OCH_2$-, $R^7R^8N-(CH_2)_a-CHR^{10}$-, $R^{11}SO_m-CH_2$-, CN oder eine freie oder funktionell abgewandelte CHO-Gruppe,

$R^3$ und $R^5$ jeweils $R^{12}$CO-, $R^{11}SO_2$ , $(AO)_2PO$-, $NO_2$ oder CN,

$R^4$ 4-$R^{13}$-5-$R^{16}$-2-thiazolyl oder 4-$R^{13}$-5-$R^{16}$-2-thiazolylamino,

$R^7$ H, A, Ar, AOOC-, Ar-alkyl-OOC-, $R^{10}$NHCO-, $R^{11}SO_2$-oder Ac,

$R^8$ H, A oder Ar-alkyl,

$R^7$ und $R^8$ zusammen auch Z, $-COCH_2CH_2CO-$, -COCH=CHCO-oder $-CO-(o-C_6H_4)-CO-$,

$R^9$ H, A, Ar, Ar-alkyl, AO-alkyl, $R^7R^8$N-alkyl, Ac, $R^{10}$NHCO-, $R^{11}SO_2$ oder $CF_3SO_2$-,

$R^{10}$ H, A oder Ar,

$R^7$ und $R^{10}$ zusammen auch Alkylen mit 2-4 C-Atomen,

$R^{11}$ A oder Ar,

$R^{12}$ HO, AO, $R^{14}$-alkyl-O-, Z=CH-O-, A, Ar, Het, $R^7R^8$N -oder $R^{15}$O-alkyl-NH-,

$R^9$ und $R^{12}$ sowie $R^8$ und $R^{12}$ jeweils zusammen auch eine Bindung,

$R^{13}$ und $R^{16}$ jeweils H, A, AOOC, $AOOCCH_2$, $H_2NCO$, ANHCO, $A_2NCO$, HOOC, Ar-alkyl, Het oder eine Phenylgruppe, die ein-bis dreifach substituiert sein kann durch A, AO, $ASO_m$-, Hal, $CF_3$, HO, $O_2N$, $R^7R^8N$, CN, $H_2NCO$, $H_2NSO_2$, $CHF_2$-O-, $R^7R^8$N-alkyl-O-, Ar oder ArO,

$R^{14}$ AO, ArO, Ar-alkyl-O-, $R^7R^8$N-, $R^{11}SO_2$O-oder $(AO)_2PO$-O-,

$R^{15}$ H, A, Ar oder Ac,

a und m jeweils 0, 1 oder 2,

A einen geradkettigen oder verzweigten, gesättigten oder ungesättigten aliphatischen oder cycloaliphatischen Kohlenwasserstoffrest mit 1-20 C-Atomen,

Ac $R^{10}$CO-, Ar-alkyl-CO-oder Ar-alkenyl-CO-,

Ar Phenyl; ein-bis dreifach durch A, AO, AcO, Hal, $CF_3$, HO, $O_2N$, $H_2N$, ANH, $A_2N$, AcNH, AOOCNH-, Ar-alkyl-OOCNH-, CN, $H_2NCO$, HOOC, AOOC, $H_2NSO_2$ und/oder $R^{11}$NHCONH-substituiertes Phenyl; oder Naphthyl,

Hal F, Cl, Br oder J,

Het einen 5-oder 6-gliedrigen ein-oder mehrkernigen heterocyclischen Rest mit 1-4 O-, N-und/oder S-Atomen, der ein-oder mehrfach durch A, AO, Hal, $CF_3$, HO, $O_2N$, $H_2N$, NHA, $NA_2$, AcNH, $ASO_m$, AOOC, CN, $H_2NCO$, HOOC, $H_2NSO_2$, $ASO_2NH$, Ar, Ar-alkenyl und/oder Pyridyl substituiert sein kann, oder 1-$R^1$-2-$R^2$-3-$R^3$-5-$R^5$-6-$R^6$-1,4-dihydro-4-pyridyl,

Z eine Alkylenkette mit 4 oder 5 C-Atomen, die durch O, HN, AN, ArN, Ar-alkyl-N, $Ar_2$CHN oder AcN unterbrochen sein kann und

-alkyl-bzw. -alkenyl-Alkylen-bzw. Alkenylen ketten mit jeweils 1-4 C-Atomen bedeuten,

sowie deren Salze.

Der Erfindung lag die Aufgabe zugrunde, neue Verbindungen mit wertvollen Eigenschaften aufzufinden, insbesondere solche, die zur Herstellung von Arzneimitteln verwendet werden können. Diese Aufgabe wurde durch die Bereitstellung der Verbindungen der Formel I gelöst.

Es wurde gefunden, daß die Verbindungen der Formel I bei guter Verträglichkeit wertvolle pharmakologische Eigenschaften besitzen. Vor allem beeinflussen sie den Calcium-Influx in die Zelle. So zeigen sich insbesondere calciumantagonistische und somit kreislaufbeeinflussende, vasodilatierende, blutdrucksenkende, antiarrhythmische und herzentlastende Wirkungen.

Die Substanzen vermindern den an der Carotisschlinge des wachen mischrassigen Hundes gemessenen Blutdruck (Methodik vgl. E.C. van Leersum, Pflügers Archiv 142, 377-395, 1911) bei nephrogen hypertonen Tieren (Methodik vgl. I.H. Page, Science 89, 273-274, 1939) im 10-Tage-Dauerversuch bei oraler Gabe von Dosen, die niedriger als 2 mg/kg/Tier liegen können, dosisabhängig auf ein erniedrigtes Niveau.

Weiterhin wird der bei kathetertragenden (Methode vgl. J.R. Weeks und J.A. Jones, Proc.Soc.Exptl.Biol.Med. 104 646-648, 1960) wachen spontan hypertonen Ratten (Stamm SHR/NIH/-MO/CHB-EMD) direkt gemessene arterielle Blutdruck nach intragastraler Gabe dosisabhängig gesenkt. Auch an der narkotisierten Katze können die blutdrucksenkenden Wirkungen nach intravenöser Applikation durch direkte Messung des Carotisdruckes verifiziert werden.

Die herzentlastende Wirkung läßt sich aus der am narkotisierten Hund nach intravenöser Gabe beobachteten Zunahme der gesamt-vaskulären Kapazität (H. Suga et al., Pflügers Archiv 361, 95-98, 1975) ableiten.

Einige Verbindungen, insbesondere solche mit $R^3$ = $NO_2$ oder $R^2$ + $R^3$ = $-CH_2$-O-CO-, wirken calciumagonistisch und somit vasokonstriktorisch, blutdrucksteigernd und positiv-inotrop; außerdem können sie den Blutzuckerspiegel senken. Dies läßt sich mit der Glucose-DH-Methode nachweisen (vgl. W. Hohenwallner und E. Wimmer, Ärztl.Lab. 22, 213-218, 1976).

Weiterhin treten antithrombotische, thrombozytenaggregationshemmende und die Erythrozytenform beeinflussende Eigenschaften auf, außerdem antiallergische, antiasthmatische, mikrozirkulationsfördernde, spasmolytische, diuretische, antimetastatische, zytoprotektive, antiatherosklerotische, endokrinologische - (z.B. die Aldosteron-Sekretion beeinflussende), antidepressive und antiinflammatorische Wirkungen, die ebenfalls nach hierfür geläufigen Methoden ermittelt werden können.

Die Verbindungen der Formel I können daher als Arzneimittel in der Human-und Veterinärmedizin verwendet werden. Ferner können sie als Zwischenprodukte zur Herstellung weiterer Arzneimittelwirkstoffe verwendet werden.

Gegenstand der Erfindung sind die 1,4-Dihydropyridine der Formel I.

Der Einfachheit halber wird im folgenden der durch $R^4$ substituierte Phenylrest als Q, der 1-$R^1$-2-$R^2$-5-$R^5$-6-$R^6$-1,4-dihydro-4-pyridylrest als DHP bezeichnet; die Formel I kann dementsprechend kurz auch Q-DHP geschrieben werden.

In den genannten Resten ist A vorzugsweise eine Alkylgruppe, die vorzugsweise 1 -12, insbesondere 1 -8, im einzelnen bevorzugt 1, 2, 3 oder 4 C-Atome hat und bevorzugt für Methyl, ferner bevorzugt für Ethyl, Propyl, Isopropyl, Butyl, Isobutyl, sek.-Butyl, tert.-Butyl, Pentyl, Isopentyl (3-Methylbutyl), Hexyl, Isohexyl (4-Methylpentyl), Heptyl, Octyl, Nonyl, Decyl, Undecyl oder Dodecyl steht, ferner auch z.B. für 1-oder 2-Methylbutyl, 1,1-, 1,2-oder 2,2-Dimethylpropyl (Neopentyl), 1-Ethylpropyl, 1-, 2-oder 3-Methylpentyl, 1-oder 2-Ethylbutyl, 1,1-, 1,2-, 1,3-, 2,2-, 2,3-oder 3,3-Dimethylbutyl, 1-Methyl-1-ethylpropyl, 1-Ethyl-2-methylpropyl, 1,1,2-oder 1,2,2-Trimethylpropyl. Die Gruppe A kann bevorzugt aber auch bedeuten: Alkenyl, insbesondere mit 2 -8 C-Atomen, z.B. Vinyl, Allyl, 1-Propenyl, 1-, 2-oder 3-Butenyl; Alkinyl, insbesondere mit 2 -8 C-Atomen, z.B. Ethinyl, Propargyl, 1-Propinyl, 1-, 2-oder 3-Butinyl; Cycloalkyl, insbesondere mit 3 -9 C-Atomen, z.B. Cyclopropyl, Cyclobutyl, Cyclopentyl, Cyclohexyl, Cycloheptyl, 1-, 2-, 3-oder 4-Methylcyclohexyl, 1-, 2-, 3-oder 4-Propylcyclohexyl; Cycloalkenyl, insbesondere mit 5 -7 C-Atomen, z.B. 1-oder 2-Cyclopentenyl, 1-, 2-oder 3-Cyclohexenyl; Cycloalkylalkyl, insbesondere mit 4 -10 C-Atomen, z.B. Cyclopropylmethyl, 1-oder 2-Cyclopropylethyl, Cyclobutylmethyl, Cyclopentylmethyl, 1-oder 2-Cyclopentylethyl, Cyclohexylmethyl, 1-oder 2-Cyclohexylethyl, 1-, 2-oder 3-Cyclohexylpropyl, Cycloheptylmethyl.

Dementsprechend ist AO vorzugsweise Methoxy oder Ethoxy. AO-alkyl ist bevorzugt Methoxymethyl, Ethoxymethyl, 2-Methoxyethyl oder 2-Ethoxyethyl. $(AO)_2PO$-ist bevorzugt Dimethylphosphonyl oder Diethylphosphonyl. AOOC ist bevorzugt Methoxycarbonyl oder Ethoxycarbonyl.

AS ist bevorzugt Methylthio oder Ethylthio. A-SO ist bevorzugt Methylsulfinyl oder Ethylsulfinyl. $A-SO_2$-ist bevorzugt Methylsulfonyl oder Ethylsulfonyl. ANH ist bevorzugt Methylamino oder Ethylamino. $A_2N$ ist bevorzugt Dimethylamino oder Diethylamino. $ASO_2NH$ ist bevorzugt Methylsulfonamido oder Ethylsulfonamido, Ac ist H-CO-, A-CO-, Ar-CO-, Ar-alkyl-CO-oder Ar-alkenyl-CO-, vorzugsweise Formyl, Acetyl, Propionyl, Butyryl, Isobutyryl, Benzoyl, Phenylacetyl oder Cinnamoyl, ferner bevorzugt Valeryl, Trimethylacetyl, Capronyl, tert.-Butylacetyl, Heptanoyl, Octanoyl, o-, m-oder p-Toluyl, o-, m-oder p-Methoxybenzoyl, o-, m-oder p-Acetoxybenzoyl, o-, m-oder p-Fluorbenzoyl, o-, m-oder p-Chlorbenzoyl, Dichlorbenzoyl wie 2,4-Dichlorbenzoyl, o-, m-oder p-Brombenzoyl, o-, m-oder p-Jodbenzoyl, o-, m-oder p-Trifluormethylbenzoyl, o-, m-oder p-Hydroxybenzoyl, o-, m-oder p-Nitrobenzoyl, o-, m-oder p-Tolylacetyl-, o-, m-oder p-Methoxyphenylacetyl, o-, m-oder p-Acetoxyphenylacetyl, o-, m-oder p-Fluorphenylacetyl, o-, m-oder p-Chlorphenylacetyl, Dichlorphenylacetyl wie 2,4-Dichlorphenylacetyl, o-, m-oder p-Methylcinnamoyl, o-, m-oder p-Methoxycinnamoyl, o-, m-oder p-Fluorcinnamoyl, o-, m-oder p-Chlorcinnamoyl, Dichlorcinnamoyl wie 2,4-Dichlorcinnamoyl.

Ar ist bevorzugt Phenyl oder einfach substituiertes Phenyl, insbesondere o-, m-oder p-Tolyl, o-, m-oder p-Ethylphenyl, o-, m-oder p-Propylphenyl, o-, m-oder p-Isopropylphenyl, o-, m-oder p-Butylphenyl, o-, m-oder p-Isobutylphenyl, o-, m-oder p-Hexylphenyl, o-, m-oder p-Methoxyphenyl, o-, m-oder p-Ethoxyphenyl, o-, m-oder p-Propoxyphenyl, o-, m-oder p-Isopropoxyphenyl, o-, m-oder p-Butoxyphenyl, o-, m-oder p-Iso butoxyphenyl, o-, m-oder p-Hexoxyphenyl, o-, m-oder p-Fluorphenyl, o-, m-oder p-Chlorphenyl, o-, m-oder p-Bromphenyl, o-, m-oder p-Jodphenyl, o-, m-oder p-Trifluormethylphenyl, o-, m-oder p-Hydroxyphenyl, o-, m-oder p-Nitrophenyl, o-, m-oder p-Aminophenyl, o-, m-oder p-Methylaminophenyl, o-, m-oder p-Ethylaminophenyl, o-, m-oder p-Dimethylaminophenyl, o-, m-oder p-Methylethylaminophenyl, o-, m-oder p-Diethylaminophenyl, o-, m-oder p-Formamidophenyl, o-, m-oder p-Acetamidophenyl, o-, m-oder p-Methoxycarbonylaminophenyl, o-, m-oder p-Ethoxycarbonylaminophenyl, o-, m-oder p-Cyanphenyl, o-, m-oder p-Carbamoylphenyl, o-, m-oder p-Carboxyphenyl, o-, m-oder p-Methoxycarbonylphenyl, o-, m-oder p-Ethoxycarbonylphenyl, o-, m-oder p-Aminosulfonylphenyl, o-, m-oder p-Methylureidophenyl, o-, m-oder p-Ethylureidophenyl, o-, m-oder p-Phenylureidophenyl.

Ar kann auch eine zweifach, ferner eine dreifach substituierte Phenylgruppe sein, worin die Substituenten gleich oder voneinander verschieden sein können, z. B. 2,3-, 2,4-, 2,5-, 2,6-, 3,4-oder 3,5-Dimethylphenyl, 2,3-, 2,4-, 2,5-, 2,6-, 3,4-oder 3,5-Dimethoxyphenyl, 2,3-, 2,4-, 2,5-, 2,6-, 3,4-oder 3,5-Difluorphenyl, 2,3-, 2,4-, 2,5-, 2,6-, 3,4-oder 3,5-Dichlorphenyl, 2,3-, 2,4-, 2,5-, 2,6-, 3,4-oder 3,5-Dihydroxyphenyl, 3,4-Diaminophenyl, 3-Methoxy-4-hydroxyphenyl, 3-Hydroxy-4-methoxyphenyl, 3-Nitro-4-hydroxyphenyl, 3-Amino-4-hydroxyphenyl, 3,4,5-Trimethoxyphenyl. Ferner kann Ar auch 1-oder 2-Naphthyl bedeuten.

Ein besonders bevorzugter Rest Ar ist Phenyl. Dementsprechend bedeutet Ar-alkyl vorzugsweise Benzyl, 1-oder 2-Phenylethyl, ArO-alkyl ist bevorzugt Phenoxymethyl, 1-oder 2-Phenoxyethyl, Ar-alkyl-O-alkyl ist bevorzugt Benzyloxymethyl, 2-Benzyloxyethyl, 2-Phenylethoxymethyl oder 2-(2-Phenylethoxy)-ethyl. Ar-alkyl-OOC-ist bevorzugt Benzyloxycarbonyl oder 2-Phenylethoxycarbonyl. Ar-alkenyl ist bevorzugt Styryl (= 2-Phenylvinyl), ferner Styrylmethyl (= 3-Phenyl-2-propen-1-yl). Ar-alkyl-O-ist bevorzugt Benzyloxy, 1-oder 2-Phenylethoxy.

Het ist vorzugsweise 2-oder 3-Furyl, 2-oder 3-Thienyl, 1-, 2-oder 3-Pyrryl, 1-, 2-, 4-oder 5-Imidazolyl, 1-, 3-, 4-oder 5-Pyrazolyl, 2-, 4-oder 5-Oxazolyl, 3-, 4-oder 5-Isoxazolyl, 2-, 4-oder 5-Thiazolyl, 3-, 4-oder 5-Isothiazolyl, 2-, 3-oder 4-Pyridyl, 2-, 4-, 5-oder 6-Pyrimidyl, weiterhin bevorzugt 1,2,3-Triazol-1-, -4-oder -5-yl, 1,2,4-Triazol-1-, -3-oder -5-yl, 1-oder 5-Tetrazolyl, 1,2,3-Oxadiazol-4-oder -5-yl, 1,2,4-Oxadiazol-3-oder -5-yl, 1,3,4-Thiadiazol-2-oder -5-yl, 1,2,4-Thiadiazol-3-oder -5-yl, 2,1,5-Thiadiazol-3-oder -4-yl, 2-, 3-, 4-, 5-oder 6-2H-Thiopyranyl, 2-, 3-oder 4-4H-Thiopyranyl, 3-oder 4-Pyridazinyl, Pyrazinyl, 2-, 3-, 4-, 5-, 6 oder 7-Benzofuryl, 2-, 3-, 4-, 5-, 6-oder 7-Benzothienyl, 1-, 2-, 3-, 4-, 5-, 6-oder 7-Indolyl, 1-, 2-, 3-, 4-, 5-, 6-oder 7-Isoindolyl, 1-, 2-, 4-oder 5-Benzimidazolyl, 1-, 3-, 4-, 5-, 6-oder 7-Benzopyrazolyl, 2-, 4-, 5-, 6-oder 7-Benzoxazolyl, 3-, 4-, 5-, 6-oder 7-Benzisoxazolyl, 2-, 4-, 5-, 6-oder 7-Benzthiazolyl, 2-, 4-, 5-, 6-oder 7-Benzisothiazolyl, 4-, 5-, 6-oder 7-Benz-2,1,3-oxadiazolyl, 2-, 3-, 4-, 5-, 6-, 7-, oder 8-Chinolyl, 1-, 3-, 4-, 5-, 6-, 7-oder 8-Isochinolyl, 1-, 2-, 3-, 4-oder 9-Carbazolyl, 1-, 2-, 3-, 4-, 5-, 6-, 7-, 8-oder 9-Acridinyl, 3-, 4-, 5-, 6-, 7-oder 8-Cinnolyl, 2-, 4-, 5-, 6-, 7-oder 8-Chinazolyl, 2-, 3-, 5-, 6-, 7-oder 8-Chinoxalyl, 1-, 2-, 5-, 6-oder 7-1H-Imidazo[4,5-b]pyridyl, 1-, 2-, 4-, 6-oder 7-1H-Imidazo[4,5-c]pyridyl, 2-, 6-, 8-oder 9-Puryl. Die heterocyclischen Reste konnen auch teilweise oder vollständig hydriert sein. Het kann also z. B. auch bedeuten 2,3-Dihydro-2-, -3-, -4-oder -5-furyl, 2,5-Dihydro-2-, -3-, -4-oder 5-furyl, Tetrahydro-2-oder -3-furyl, Tetrahydro-2-oder -3-thienyl, 2,3-Dihydro-1-, -2-, -3-, -4-oder -5-pyrryl, 2,5-Dihydro-1-, -2-, -3-, -4-oder -5-pyrryl, 1-, 2-oder 3-Pyrrolidinyl, Tetrahydro-1-, -2-oder -4-imidazolyl, 2,3-Dihydro-1-, -2-, -3-, -4-oder 5-pyrazolyl, 2,5-Dihydro-1-, -2-, -3-, -4-oder 5-pyrazolyl, Tetrahydro-1-, -3-, oder -4-pyrazolyl, 1,4-Dihydro-1-, -2-, -3-oder -4-pyridyl, 1,2,3,4-Tetrahydro-1-, 2-, -3-, -4-, -5-oder -6-pyridyl, 1,2,3,6-Tetrahydro-1-, -2-, -3-, -4-, -5-oder -6-pyridyl, 1-, 2-, 3-oder 4-Piperidinyl, 2-, 3-oder 4-Morpholinyl, Tetrahydro-2-, -3-oder -4-pyranyl, 1,4-Dioxanyl, 1,3-Dioxan-2-, -4-oder -5-yl, Hexahydro-1-, -3-oder -4-pyridazinyl, Hexahydro-1-, -2-, -4-oder -5-pyrimidyl, 1-, 2-oder 3-Piperazinyl, 1,2,3,4-Tetrahydro-1-, -2-, -3-, -4-, -5-, -6-, -7-, oder -8-chinolyl, 1,2,3,4-Tetrahydro-1-, -2-, -3-, -4-, -5-, -6-, -7-oder 8-isochinolyl.

Die heterocyclischen Reste können auch wie angegeben substituiert sein. Het kann also z.B. auch bedeuten: 3-, 4-oder 5-Methyl-2-furyl, 2-, 4-oder 5-Methyl-3-furyl, 2,4-Dimethyl-3-furyl, 5-Nitro-2-furyl, 5-Styryl-2-furyl, 3-, 4-oder 5-Methyl-2-thienyl, 2-, 4-oder 5-Methyl-3-thienyl, 3-Methyl-5-tert.-butyl-2-thienyl, 5-Chlor-2-thienyl, 5-Phenyl-2-oder -3-thienyl, 1-, 3-, 4-oder 5-Methyl-2-pyrryl, 1-Methyl-4-oder -5-nitro-2-pyrryl, 3,5-Dimethyl-4-ethyl-2-pyrryl, 4-Methyl-5-pyrazolyl, 4-oder 5-Methyl-2-thiazolyl, 2-oder 5-Methyl-4-thiazolyl, 2-oder 4-Methyl-5-thiazolyl, 2,4-Dimethyl-5-thiazolyl, 3-, 4-, 5-oder 6-Methyl-2-pyridyl, 2-, 4-, 5-oder 6-Methyl-3-pyridyl, 2-oder 3-Methyl-4-pyridyl, 3-, 4-, 5-oder 6-Chlor-2-pyridyl, 2-, 4-, 5-oder 6-Chlor-3-pyridyl, 2-oder 3-Chlor-4-pyridyl, 2,6-Dichlorpyridyl, 5-oder 6-Methyl-4-pyrimidyl, 2,6-Dihydroxy-4-pyrimidyl, 5-Chlor-2-methyl-4-pyrimidyl, 3-Methyl-2-benzofuryl, 2-Ethyl-3-benzofuryl, 7-Methyl-2-benzothienyl, 1-, 2-, 4-, 5-, 6-oder 7-Methyl-3-indolyl, 1-Methyl-5-oder -6-benzimidazolyl, 1-Ethyl-5-oder -6-benzimidazolyl, 3-, 4-, 5-, 6-, 7-oder 8-Hydroxy-2-chinolyl.

Z bedeutet vorzugsweise Tetramethylen, Pentamethylen, 3-Oxapentamethylen, 3-Aza-, 3-A-aza-wie 3-Methyl-aza-, 3-Ac-aza-wie 3-Acetyl-aza-, 3-Ar-aza-wie 3-Phenyl-aza-, 3-Ar-alkyl-aza-wie 3-Benzyl-aza-oder 3-$Ar_2CH$-aza-wie 3-Diphenylmethyl-aza-pentamethylen.

Im einzelnen bedeutet $R^1$ vorzugsweise H, in zweiter Linie bevorzugt Methyl, Ethyl, Benzyl, 2-Methoxyethyl, 2-Phenoxyethyl, 2-Benyloxyethyl, 2-Dimethylaminoethyl, 2-Diethylaminoethyl, 2-Pyrrolidinoethyl, 2-Piperidinoethyl oder 2-Morpholinoethyl.

$R^2$ und $R^6$ sind gleich oder verschieden und bedeuten vorzugsweise jeweils A, insbesondere Alkyl mit 1 -8 C-Atomen oder Cycloalkyl mit 3 -7 C-Atomen, bevorzugt Methyl, ferner bevorzugt H, Phenyl, Benzyl, Chlormethyl, Brommethyl, Hydroxymethyl oder 2-Aminoethoxymethyl; besonders bevorzugt sind $R^2$ und $R^6$ gleich und bedeuten Methyl.

$R^3$ und $R^5$ sind gleich oder verschieden und bedeuten vorzugsweise jeweils $R^{12}CO$-, insbesondere AOOC (worin A vorzugsweise Alkyl mit 1 -8 C-Atomen, bevorzugt Methyl, Ethyl oder Isopropyl, oder Cycloalkyl mit 3 -7 C-Atomen bedeutet), ferner 2-Alkoxyethoxycarbonyl (worin Alkoxy vorzugsweise 1 -4 C-Atome hat), insbesondere 2-Methoxyethoxycarbonyl, 2-Ethoxyethoxycarbonyl oder 2-Propoxyethoxycarbonyl, 2-N,N-Dialkylaminoethoxycarbonyl (worin die Alkylgruppen vorzugsweise jeweils 1 - 4 C-Atome enthalten) oder 2-N-Alkyl-N-Aralkylaminoethoxycarbonyl (worin die Alkylgruppe vorzugsweise 1 -4, die Aralkylgruppe vorzugsweise 7 -10 C-Atome enthält) wie 2-N-Methyl-N-benzylamino-ethoxycarbonyl. Ferner kann eine der Gruppen $R^3$ oder $R^5$ bevorzugt $NO_2$ oder CN bedeuten.

Die Gruppen $R^2$ und $R^3$ zusammen können bevorzugt auch -$CH_2$-O-CO-bedeuten und so zusammen mit dem 1,4-Dihydropyridinring ein 5-Oxo-1,4,5,7-tetrahydrofuro[3,4-b]-pyridin bilden.

$R^4$ steht vorzugsweise in der o-Stellung, ferner bevorzugt in der m-Stellung des Phenylrings und ist vorzugsweise 4-$R^{13}$-5-$R^{16}$-2-thiazolylamino.

$R^7$ ist vorzugsweise H, A (insbesondere Alkyl mit 1 -4 C-Atomen), Ar (insbesondere Phenyl oder Methoxyphenyl) oder Ac (insbesondere Alkanoyl mit 1 -4 C-Atomen, Benzoyl oder Phenylacetyl).

$R^8$ ist vorzugsweise H, A (insbesondere Alkyl mit 1 -4 C-Atomen) oder Benzyl.

Dementsprechend ist die Gruppe $R^7R^8N$ vorzugsweise Amino, Methyl-, Ethyl-, Propyl-, Butyl-, Benzyl-, Dimethyl-, Diethyl-, Dipropyl-, Dibutyl-, N-Methyl-N-benzyl-oder N-Methyl-N-p-methoxybenzylamino, Formamido, Acetamido, Propionamido, Benzamido, N-Methyl-formamido oder N-Methyl-acetamido.

$R^7$ und $R^8$ sind zusammen mit dem N-Atom, an das sie gebunden sind, vorzugsweise auch Pyrrolidino, Piperidino, Morpholino, Piperazino, N-Methylpiperazino, N-Acetylpiperazino, N-Phenylpiperazino, N-Benzylpiperazino, N-Diphenylmethylpiperazino, Succinimido, Maleinimido oder Phthalimido.

$R^9$ ist vorzugsweise H, ferner bevorzugt Ac (insbesondere Alkanoyl mit 1 -4 C-Atomen).

$R^{10}$ ist vorzugsweise H, A (insbesondere Alkyl mit l -4 C-Atomen) oder Phenyl.

$R^{11}$ ist vorzugsweise A (insbesondere Alkyl mit 1 -4 C-Atomen) oder Phenyl.

$R^{12}$ ist vorzugsweise AO (insbesondere Alkoxy mit 1 -4 C-Atomen), ferner vorzugsweise AO-alkoxy-(insbesondere Alkoxyethoxy mit 3 -6 C-Atomen oder $R^7R^8$N-alkoxy-(insbesondere Dialkylaminoalkoxy mit 4 -10 C-Atomen oder N-Alkyl-N-Ar-alkyl-aminoalkoxy mit 10 -18 C-Atomen).

$R^{13}$ und $R^{16}$ sind vorzugsweise Phenyl; monosubstituiertes Phenyl, wobei der Substituent vorzugsweise in p-Stellung steht, bevorzugt Alkylphenyl wie o-, m-oder p-Tolyl, o-, m-oder p-Ethylphenyl, o-, m-oder p-tert.-Butylphenyl; Alkoxyphenyl wie o-, m-oder p-Methoxyphenyl, o-, m-oder p-Ethoxyphenyl; Alkylthiophenyl wie o-, m-oder p-Methylthiophenyl; Alkylsulfinylphenyl wie o-, m-oder p-Methylsulfinylphenyl; Alkylsulfonylphenyl wie o-, m-, oder p-Methylsulfonylphenyl; Halogenphenyl wie o-, m-oder p-Fluorphenyl, o-, m-oder p-Chlorphenyl, o-, m-oder p-Bromphenyl, o-, m-oder p-Jodphenyl; o-, m-oder p-Trifluormethylphenyl; o-, m-oder p-Hydroxyphenyl; o-, m-oder p-Nitrophenyl; o-, m-oder p-Aminophenyl; Alkylaminophenyl wie o-, m-oder p-Methylaminophenyl; Dialkylaminophenyl wie o-, m-oder p-Dimethylaminophenyl, o-, m-oder p-Diethylaminophenyl; o-, m-oder p-Pyrrolidinophenyl; o-, m-oder p-Piperidinophenyl; o-, m-oder p-Morpholinophenyl; o-, m-oder p-Piperazinophenyl; 4-Alkylpiperazinophenyl wie o-, m-oder p-4-Methylpiperazinophenyl; 4-Acylpiperazinophenyl wie o-, m-oder p-4-Acetylpiperazinophenyl; 4-Arylpiperazinophenyl wie o-, m-oder p-4-Phenylpiperazinophenyl, o-, m-oder -p-4-(o-Methoxyphenyl)-piperazinophenyl, o-, m-oder p-4-(p-Methoxyphenyl)-piperazinophenyl; 4-Ar-alkylpiperazinophenyl wie o-, m-oder p-4-Benzylpiperazinophenyl; 4-Diarylmethylpiperazinophenyl wie o-, m-oder p-4-Diphenylmethylpiperazinophenyl; Alkanoylaminophenyl wie o-, m-oder p-Acetamidophenyl; Aroylaminophenyl wie o-, m-oder p-Benzamidophenyl, o-, m-oder p-(o-, m-oder p-Toluylamido)-phenyl, o-, m-, oder p-(o-, m-oder p-Methoxybenzamido)-phenyl, o-, m-oder p-(o-, m-oder p-Chlorbenzamido)-phenyl, o-, m-oder p-(2,4-Dichlorbenzamido)-phenyl; Alkoxycarbonylaminophenyl wie o-, m-oder p-Ethoxycarbonylaminophenyl; o-, m-oder p-Phthalimidophenyl; o-, m-oder p-Isoindolinophenyl; o-, m-oder p-Ureidophenyl; N'-Alkylureidophenyl wie o-, m-oder p-N'-Methylureidophenyl; N'-Arylureidophenyl wie o-, m-oder p-N'-Phenylureidophenyl; o-, m-oder p-Cyanphenyl; o-, m-oder p-Carbamoylphenyl; o-, m-oder p-Aminosulfonylphenyl; o-, m-oder p-Difluormethoxyphenyl; o-, m-oder p-2,2-Difluorethoxyphenyl; o-, m-oder p-Dialkylaminoalkoxyphenyl wie o-, m-oder p-2-Dimethylaminoethoxyphenyl, o-, m-oder p-2-Diethylaminoethoxyphenyl; Ar-phenyl wie 2-, 3-oder 4-Biphenylyl; Aryloxyphenyl wie o-, m-oder p-Phenoxyphenyl; zweifach substituiertes Phenyl, bevorzugt 2,3-, 2,4-, 2,5-, 2,6-, 3,4-oder 3,5-Dimethylphenyl, -Dimethoxyphenyl, -Difluorphenyl, -Dichlorphenyl oder -Dihydroxyphenyl, 3-Methoxy-4-hydroxyphenyl, 3-Hydroxy-4-methoxyphenyl, 3-Nitro-4-methoxyphenyl, 3-Acetamido-4-methoxyphenyl; dreifach substituiertes Phenyl, bevorzugt 2,3,4-, 2,3,5-, 2,3,6-, 2,4,5-, 2,4,6-oder 3,4,5-Trimethylphenyl, -Trimethoxyphenyl oder -Trifluorphenyl, 2,6-Dimethyl-4-tert.-butylphenyl, 2,4-Dimethoxy-5-bromphenyl; Het wie bevorzugt 2-oder 3-Furyl, 2-oder 3-Thienyl, 1-, 2-oder 3-Pyrryl, 1-Methyl-2-pyrryl, 2-, 3-oder 4-Pyridyl, 3-Cyan-6-methyl-2-oxo-1,2-dihydro-5-pyridyl oder einen der übrigen oben für Het als bevorzugt genannten Reste; weiterhin Wasserstoff; Alkyl mit vorzugsweise 1 -4 C-Atomen wie Methyl, Ethyl, Propyl, Isopropyl, Butyl, Isobutyl, sek.-Butyl oder tert.-Butyl; Alkoxycarbonyl wie Methoxycarbonyl oder Ethoxycarbonyl; Alkoxycarbonylmethyl wie Methoxycarbonylmethyl oder Ethoxycarbonylmethyl; Carbamoyl; N-Alkylcarbamoyl wie N-Methylcarbamoyl, N-Ethylcarbamoyl; N,N-Dialkylcarbamoyl wie N,N-Dimethylcarbamoyl, N,N-Diethylcarbamoyl; Carboxy; Ar-alkyl wie Benzyl, 1-oder 2-Phenylethyl.

Besonders bevorzugt ist $R^{13}$ Phenyl, o-, m-oder p-Tolyl, o-, m-oder p-Methoxyphenyl, o-, m-oder p-Fluorphenyl, o-, m-oder p-Chlorphenyl, o-, m-oder p-Trifluormethylphenyl oder 3,4-Dichlorphenyl, und $R^{16}$ ist bevorzugt H, ferner Methyl, Phenyl oder Benzyl.

$R^{14}$ ist vorzugsweise AO mit 1 -4 C-Atomen wie Methoxy, Ethoxy, Propoxy oder Butoxy, ferner bevorzugt $R^7R^8$N.

$R^{15}$ ist vorzugsweise H oder A, insbesondere Alkyl mit 1 -4 C-Atomen.

Die Parameter a und m sind vorzugsweise 0 oder 1.

Dementsprechend sind Gegenstand der Erfindung insbesondere diejenigen Verbindungen der Formel I, in denen mindestens einer der genannten Reste oder Parameter eine der vorstehend angegebenen bevorzugten Bedeutungen hat.

Einige bevorzugte Gruppen von Verbindungen können durch die folgenden Teilformeln Ia bis Ii ausgedrückt werden, die der Formel I entsprechen und worin die nicht näher bezeichneten Reste die bei der Formel I angegebenen Bedeutungen haben, worin jedoch in

Ia $R^4$ 4-$R^{13}$-5-$R^{16}$-2-thiazolyl bedeutet;

Ib $R^4$ 4-$R^{13}$-5-$R^{16}$-2-thiazolyl,
$R^{13}$ eine Phenylgruppe, die ein-bis dreifach substituiert sein kann durch A, AO, $ASO_m$-, Hal, $CF_3$, HO, $O_2N$, $R^7R^8N$, CN, $H_2NCO$, $H_2NSO_2$, $CHF_2$-O-, $R^7R^8N$-alkyl-O-, Ar oder ArO und
$R^{16}$ H bedeutet;

Ic $R^4$ 4-$R^{13}$-5-$R^{16}$-2-thiazolyl,
$R^{13}$ Phenyl, Tolyl, Methoxyphenyl, Chlorphenyl oder Dichlorphenyl und
$R^{16}$ H bedeutet;

Id $R^4$ 4-$R^{13}$-5-$R^{16}$-2-thiazolylamino bedeutet;

Ie $R^4$ 4-$R^{13}$-2-thiazolylamino bedeutet;

If $R^4$ 4-$R^{13}$-5-$R^{16}$-2-thiazolylamino,
$R^{13}$ H, A, AOOC, $AOOCCH_2$, Het oder eine Phenylgruppe, die ein-bis dreifach substituiert sein kann durch A, AO, $ASO_m$-, Hal, $CF_3$, HO, $O_2N$, $R^7R^8N$, CN, $H_2NCO$, $H_2NSO_2$, $CHF_2$-O-, $R^7R^8N$-alkyl-O-, Ar oder ArO und
$R^{16}$ H, A, Benzyl oder eine Phenylgruppe, die ein-bis dreifach substituiert sein kann durch A, AO, $ASO_m$-, Hal, $CF_3$, HO, $O_2N$, $R^7R^8N$, CN, $H_2NCO$, $H_2NSO_2$, $CHF_2$-O-, $R^7R^8N$-alkyl-O-, Ar oder ArO bedeuten;

Ig $R^4$ 4-$R^{13}$-5-$R^{16}$-2-thiazolylamino,
$R^{13}$ H, A, AOOC, $AOOCCH_2$, Pyridyl, 3-Cyan-6-methyl-2-pyridon-5-yl oder eine Phenylgruppe, die ein-bis dreifach substituiert sein kann durch A, AO, Hal, $CF_3$, HO, $O_2N$, A-CO-NH, CN, $H_2NSO_2$ oder Phenyl und
$R^{16}$ H, A, Benzyl oder Phenyl bedeutet;

Ih $R^4$ 4-$R^{13}$-5-$R^{16}$-2-thiazolylamino,
$R^{13}$ eine Phenylgruppe, die einfach durch $CH_3$, $CH_3O$, F, Cl oder $CF_3$ oder zweifach durch Cl substituiert sein kann und
$R^{16}$ H bedeuten;

Ii $R^4$ 4-$R^{13}$-5-$R^{16}$-2-thiazolylamino,
$R^{13}$ Phenyl, p-Tolyl p-Methoxyphenyl, p-Fluorphenyl, m-oder p-Chlorphenyl, m-Trifluormethylphenyl oder 3,4-Dichlorphenyl und
$R^{16}$ H bedeuten.

Ferner sind Verbindungen der Teilformeln I′ sowie Ia′ bis Ii′ bevorzugt, die den Formeln I sowie Ia bis Ii entsprechen, worin jeweils zusätzlich $R^2$ und $R^6$ Methylgruppen bedeuten.

Ferner sind Verbindungen der Teilformeln I″ sowie Ia″ bis Ii″ bevorzugt, die den Formeln I sowie Ia bis Ii entsprechen, worin jeweils zusätzlich $R^3$ und $R^5$ jeweils AOOC und die beiden Reste A (gleich oder verschieden!) Alkylgruppen mit 1 -4 C-Atomen bedeuten.

Insbesondere sind Verbindungen der Teilformeln I‴ sowie Ia‴ bis Ii‴ bevorzugt, die den Formeln I sowie Ia bis Ii entsprechen, worin jeweils zusätzlich
$R^2$ und $R^6$ jeweils $CH_3$,
$R^3$ und $R^5$ jeweils AOOC und
A eine Alkylgruppe mit 1 -4 C-Atomen bedeuten,
wobei, wenn mehrere Gruppen A im Molekül vorhanden sind, diese gleich oder voneinander verschieden sein können.

Von den Verbindungen der Formeln I, I′, I″, I‴, Ia bis Ii, Ia′ bis Ii′, Ia″ bis Ii″, Ia‴ bis Ii‴ sind jeweils diejenigen bevorzugt, bei denen der Rest $R^1$ H bedeutet und/oder bei denen der Rest $R^4$ in o-oder m-Stellung, insbesondere in o-Stellung steht.

Gegenstand der Erfindung ist ferner ein Verfahren zur Herstellung von Dihydropyridinen der Formel I sowie von ihren Salzen, dadurch gekennzeichnet, daß man Verbindungen der Formeln II, III, IV und V

$R^1$-$NH_2$ II
$R^2$-CO-$CH_2$-$R^3$ III
$R^6$-CO-$CH_2$-$R^5$ IV

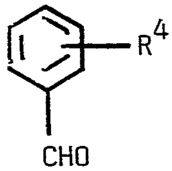

V

oder deren funktionelle Derivate, falls erwünscht stufenweise, miteinander umsetzt,

oder daß man ein Thioamid der Formel VI

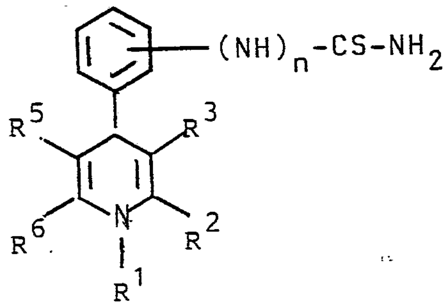

VI

worin

n O oder 1 beduetet,

mit einer Carbonylverbindung der Formel VII

$R^{16}$-CHX-CO-$R^{13}$ VII

worin

X Cl, Br, J oder eine freie oder reaktionsfähig veresterte OH-Gruppe bedeutet

umsetzt,

oder daß man eine Verbindung der Formel VIII

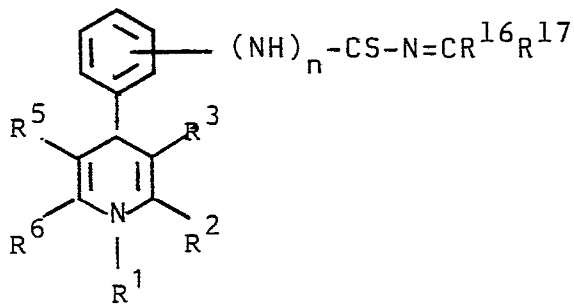

VIII

worin

$R^{17}$ HO, AO oder $A_2N$ bedeutet

mit einer Verbindung der Formel IX

$R^{13}$-$CH_2$-X IX

umsetzt,

und/oder daß man in einer Verbindung der Formel I einen oder mehrere der Reste $R^1$, $R^2$, $R^3$, $R^4$, $R^5$ und/oder $R^6$ in andere Reste $R^1$, $R^2$, $R^3$, $R^4$, $R^5$ und/oder $R^6$ umwandelt und/oder eine basische bzw. saure

Verbindung der Formel I durch Behandeln mit einer Säure bzw. Base in eines ihrer Salze überführt.

Die Herstellung der Verbindungen der Formel I erfolgt im übrigen nach an sich bekannten Methoden, wie sie in der Literatur (z.B. in Standardwerken wie Houben-Weyl, Methoden der Organischen Chemie, Georg-Thieme-Verlag, Stuttgart) beschrieben sind, und zwar unter Reaktionsbedingungen, wie sie für die genannten Umsetzungen bekannt und geeignet sind. Dabei kann man auch von an sich bekannten, hier nicht näher erwähnten Varianten Gebrauch machen.

Der Fachmann kann durch Routinemethoden entsprechende Synthesemethoden aus dem Stand der Technik entnehmen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

Andererseits ist es möglich, die Reaktion stufenweise durchzuführen. So kann man zunächst einzelne der Ausgangsstoffe II, III, IV und V miteinander umsetzen und die erhaltenen Zwischenprodukte dann mit den restlichen Ausgangsstoffen zur Reaktion bringen.

So ist es möglich, Verbindungen der Formel I nicht nur durch die Reaktion (a):

(a) II + III + IV + V

zu erhalten, sondern z. B. auch durch die Reaktionen (b) bis (d):

(b) $R^2$-CO-$CR^3$=CH-Q (= X; erhältlich aus III + V) + II + IV bzw.
$R^6$-CO-$CR^5$=CH-Q (= XI; erhältlich aus IV + V) + II + III;

(c) X + $R^1$NH-$CR^6$=$CHR^5$ (= XII; erhältlich aus II + IV) bzw.
XI + $R_1$NH-$CR^2$=$CHR^3$ (= XIII; erhältlich aus II + III);

(d) $R^2$-CO-$CHR^3$-CHQ-$CHR^5$-CO-$R^6$ (= XIV; erhältlich aus III + IV + V) + II.

An Stelle der genannten Ausgangsstoffe können auch funktionelle Derivate dieser Verbindungen in die Reaktion eingesetzt werden. Als solche eignen sich z. B. Enamine (wie XII und XIII), Enolether, z. B. Trialkylsilyl-enolether wie Trimethylsilyl-oder Dimethyl-tert.-butylsilylenolether, Tetronsäuremethylester, oder Enolester, z. B. Enolacetate, Enol-methansulfonate, Enol-p-toluolsulfonate von III und IV, Acetale oder Halbacetale von V, Salze (z. B. Hydrochloride, Sulfate, Nitrate, Acetate) von II.

Die Ausgangsstoffe der Formeln II bis V sowie X bis XIV sind literaturbekannt oder können nach literaturbekannten Methoden hergestellt werden (vgl. z. B. Houben-Weyl, 1. c.).

Typische Ausgangsstoffe der Formel II sind z. B. Ammoniak, der in gasförmigem Zustand oder in wäßriger oder nicht-wäßriger Lösung angewendet werden kann, ferner Alkylamine wie Methylamin oder Ethylamin, Aralkylamine wie 2-Methoxyethylamin oder 2-Ethoxyethylamin, Aryloxyalkylamine wie 2-Phenoxyethylamin, Aralkoxyalkylamine wie 2-Benzyloxyethylamin, Aminoalkylamine wie 2-Dimethylamino-, 2-Diethylamino-, 2-Pyrrolidino-, 2-Piperidino-, 2-Morpholino-, 2-Piperazino-, 2-(4-Methylpiperazino)-oder 2-(4-Phenylpiperazino)-ethylamin, 3-Dimethylamino-, 3-Diethylamino-, 3-Pyrrolidino-, 3-Piperidino-, 3-Morpholino-, 3-Piperazino-, 3-(4-Methylpiperazino)-oder 3-(4-Phenylpiperazino)-propylamin.

Typische Ausgangsstoffe der Formeln III und IV sind Acetessigsäuremethyl-, -ethyl-, -isopropyl-, -isobutyl-, -2-methoxyethyl-, -2-propoxyethyl-, -2-phenoxyethyl-, -2-benzyloxyethyl-, -2-dimethylaminoethyl-, oder -2-(N-benzyl-N-methylamino)-ethylester, ferner Malonaldehydsäuremethyl-, -ethyl-oder -isopropylester, 3-Oxo-4-phenylbuttersäuremethyl-, -ethyl-oder -isopropylester, 3-Oxo-4-chlorbuttersäuremethyl-, -ethyl-oder -isopropylester, 3-Oxo-4-brombuttersäuremethyl-, -ethyl-oder -isopropylester, 3-Oxo-4,4,4-trifluorbuttersäuremethyl, -ethyl-oder -isopropylester, 3-Oxo-4-hydroxybuttersäuremethyl-, -ethyl-oder -isopropylester, 3-Oxo-4-acetoxybuttersäuremethyl-, -ethyl-oder -isopropylester, 3-Oxo-4-carbamoyloxybuttersäuremethyl-, -ethyl-oder -isopropylester, 3-Oxo-4-dimethylaminobuttersäuremethyl-, -ethyl-oder -isopropylester, 3-Oxo-4-methylthiobuttersäuremethyl-, -ethyl-oder -isopropylester, 3-Oxo-4-methylsulfinylbuttersäuremethyl-, -ethyl-oder -isopropylester, 3-Oxo-4-methylsulfonylbuttersäuremethyl-, -ethyl-oder -isopropylester, 3-Oxo-3-cyanpropionsäuremethyl-, -ethyl-oder -isopropylester, 2-Oxo-bernsteinsäuredinitril, 3,4-Dioxobuttersäuremethyl-, -ethyl-oder -isopropylester, Acetylaceton, Hexan-2,5-dion, Heptan-2,5-dion, Benzoylaceton, 2-Acetoacetylthiophen, Acetoacetamid, Acetessigsäure-N-methylamid, -N,N-dimethylamid, -pyrrolidid, -piperidid, -morpholid, -2-hydroxyethylamid, -2-methoxyethylamid, -2-phenoxyethylamid, -2-acetoxyethylamid, Methylsulfonylaceton, Phenylsulfonylaceton, Dimethylphosphonylaceton, Diethylphosphonylaceton, Nitroaceton, Cyanaceton, Tetronsäure.

Typische Ausgangsstoffe der Formel V sind solche der Formeln Va und Vb, worin die Substituenten bevorzugt in o-Stellung, weiterhin bevorzugt in m-Stellung zur Aldehydgruppe stehen:

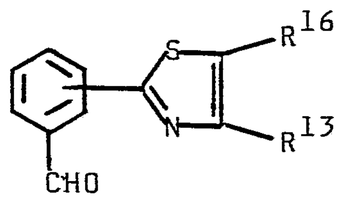 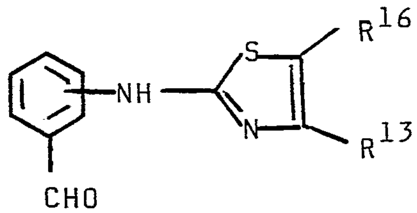

Va Vb

Unter diesen sind diejenigen der Formal Va bevorzugt, z.B. 2-(o-Formylphenyl)-4-phenylthiazol, 2-(m-Formylphenyl)-4-phenylthiazol,
2-(o-oder m-Formylphenyl)-4-o-, -m-, oder -p-tolylthiazol,
2-(o-oder m-Formylphenyl)-4-o-, -m-, oder -p-methoxyphenylthiazol,
2-(o-oder m-Formylphenyl)-4-o-, -m-, oder -p-fluorphenylthiazol,
2-(o-oder m-Formylphenyl)-4-o-, -m-, oder -p-chlorphenylthiazol,
2-(o-oder m-Formylphenyl)-4-o-, -m-, oder -p-trifluormethylphenylthiazol.

Unter den Verbindungen der Formel Vb sind z.B. bevorzugt 2-(o-oder m-Formylanilino)-4-phenylthiazol,
2-(o-oder m-Formylanilino)-4-o-, -m-oder -p-tolylthiazol,
2-(o-oder m-Formylanilino)-4-o-, -m-oder -p-methoxyphenylthiazol,
2-(o-oder m-Formylanilino)-4-o-, -m-oder -p-fluorphenylthiazol,
2-(o-oder m-Formylanilino)-4-o-, -m-oder -p-chlorphenylthiazol,
2-(o-oder m-Formylanilino)-4-o-, -m-oder -p-trifluormethylphenylthiazol.

Typische Ausgangsstoffe der Formeln X und XI sind z. B. 2-Acetyl-o-oder m-(4-phenyl-2-thiazolyl)-zimtsäuremethyl-, -ethyl-oder -isopropylester sowie die entsprechenden 4-o-, m-oder -p-Tolyl-, 4-o-, -m-oder -p-Methoxyphenyl-, 4-o-, -m-oder -p-Fluorphenyl-, 4-o-, -m-oder -p-Chlorphenyl-, 4-o-, -m-oder -p-Trifluormethylphenyl-2-thiazolylderivate.

Typische Ausgangsstoffe der Formeln XII und XIII sind z. B. solche der Formel $H_2N-C(CH_3)=C-CO-R^{12}$ wie 3-Aminocrotonsäuremethyl-, -ethyl-, -isopropyl-, -isobutyl-, -2-methoxyethyl-, 2-propoxyethyl-, -2-phenoxyethyl-, -2-benzyloxyethyl-, -2-dimethylaminoethyl-oder -2-(N-benzyl-N-methylamino)-ethylester, 3-Methylaminocrotonsäuremethyl-, -ethyl-oder -isopropylester, 3-(2-Morpholinoethylamino)-crotonsäuremethyl-, -ethyl-oder -isopropylester, ferner Tetronsäureamid.

Typische Ausgangsstoffe der Formel XIV sind z. B. 3,5-Diethoxycarbonyl-4-o-oder -m-(4-phenyl-2-thiazolyl-phenyl-2,6-heptandion sowie die entsprechenden 4-o-, -m-oder -p-Tolyl-, 4-o-, -m-oder -p-Methoxyphenyl-, 4-o-, -m-oder -p-Fluorphenyl-, 4-o-, -m-oder -p-Chlorphenyl-, 4-o-, -m-oder -p-Trifluormethylphenyl-2-thiazolyldervate.

Die oben definierten Reaktionen (a), (b), (c) und (d) erfolgen in der Regel unter den Bedingungen der Hantzsch-Synthese von 1,4-Dihydropyridinen (vgl. z. B. The Merck Index, Tenth Edition, Merck & Co., Inc., Rahway, N.J., USA, 1983, Seite ONR-39, und die dort zitierte Literatur). Die Ausgangsstoffe der Formeln III bis V sowie X bis XIV werden in der Regel in stöchiometrischen Mengen oder in geringem Über-oder Unterschuß, diejenigen der Formel II -besonders im Fall von $R^1$ = H -in geringem oder größerem Überschuß eingesetzt.

Man arbeitet dabei in Abwesenheit oder -vorzugsweise -in Anwesenheit eines inerten Lösungs-oder Verdünnungsmittels. Als Lösungsmittel eignen sich z. B. Alkohole wie Methanol, Ethanol, Propanol, Isopropanol; Ether wie Diethyl-oder Diisopropylether, Tetrahydrofuran, Dioxan, Ethylenglykol-mono-oder -dimethylether; Carbonsäuren wie Essigsäure; Nitrile wie Acetonitril; Ester wie Ethylacetat; Amide wie Dimethylformamid (DMF), Dimethylacetamid oder Phosphorsäure-hexamethyltriamid; Sulfoxide wie Dimethylsulfoxid; Kohlenwasserstoffe wie Hexan, Cyclohexan, Benzol, Toluol; chlorierte Kohlenwasserstoffe wie Dichlormethan, Chloroform, Tetrachlorkohlenstoff, Trichlorethylen oder 1,2-Dichlorethan; Amine wie Pyridin. Weiterhin eignen sich Gemische dieser Lösungsmittel untereinander sowie Gemische mit Wasser. Im allgemeinen arbeitet man bei Reaktionstemperaturen zwischen 20 und 150°, vorzugsweise 50 und 100°, insbesondere jedoch bei der Siedetemperatur des jeweiligen Lösungsmittels.

Die Umsetzung wird im allgemeinen bei Normaldruck durchgeführt; man kann aber auch bei erhöhtem Druck bis zu etwa 100 bar arbeiten insbesondere bei Verwendung niedrigsiedender Lösungsmittel.

Die Verbindungen der Formel I sind ferner durch Umsetzung von Verbindungen der Formel VI mit Verbindungen der Formel VII erhältlich. Insbesondere können Verbindungen der Formel Id aus Thioharnstoffen der Formel VI (n = 1) hergestellt werden. Diese sind ihrerseits zugänglich aus den entsprechenden Nitroverbindungen der Formel $DHP-C_6H_4-NO_2$ durch Reduktion zu den Aminen der Formel $DHP-C_6H_4-NH_2$,

Reaktion mit Benzoylisothiocyanat (zweckmäßig in situ hergestellt aus NaSCN und Benzoylchlorid in Aceton) zu den Benzoylthioharnstoffen der Formel DHP-$C_6H_4$-NH-CS-NH-$COC_6H_5$ und Abspaltung der Benzoylgruppe mit $K_2CO_3$ in wässerigem Methanol. Die Thioamide der Formel VI (n = 0) können z.B. aus den Nitrilen der Formel DHP-$C_6H_4$-CN mit $H_2S$ erhalten werden.

Typische Ausgangsstoffe der Formel VI sind 2,6-Dimethyl-3,5-dimethoxycarbonyl-4-o-und m-thioureidophenyl-1,4-dihydropyridin, 2,6-Dimethyl-3,5-diethoxycarbonyl-4-o-und -m-thioureidophenyl-1,4-dihydropyridin, 2,6-Dimethyl-3,5-dimethoxycarbonyl-4-o-und -m-aminothiocarbonylphenyl-1,4-dihydropyridin sowie 2,6-Dimethyl-3,5-diethoxycarbonyl-4-o-und -m-aminothiocarbonylphenyl-1,4-dihydropyridin.

In den -meist bekannten -Verbindungen der Formel VII bedeutet X vorzugsweise Cl oder Br, aber auch eine reaktionsfähig veresterte OH-Gruppe wie Alkylsulfonyloxy mit insbesondere 1-4 C-Atomen (z.B. Methansulfonyloxy) oder Arylsulfonyloxy mit insbesondere 6-10 C-Atomen (z.B. Benzol-, p-Toluol-, 1-oder 2-Naphthalinsulfonyloxy).

Typische Verbindungen der Formel VII sind $\alpha$-Chloracetophenon, $\alpha$-Bromacetophenon, $\alpha$-Chlor-und $\alpha$-Brom-o-, -m-und p-methylacetophenon, $\alpha$-Chlor-und $\alpha$-Brom-o-, -m-und p-methoxyacetophenon, $\alpha$-Chlor-und $\alpha$-Brom-o-, -m-und p-fluoracetophenon, $\alpha$,o-, $\alpha$,m-und a,p-Dichloracetophenon, $\alpha$-Brom-o-, -m-und -p-chloracetophenon, $\alpha$-Chlor-und $\alpha$-Brom-o-, -m-und -p-Trifluormethylacetophenon.

Die Umsetzung von VI und VII gelingt in Abwesenheit oder -vorzugsweise -in Gegenwart eines der oben genannten inerten Lösungs-oder Verdünnungsmittel oder Gemische bei Temperaturen zwischen etwa 20 und 150 °, vorzugsweise 50 und 100 °, insbesondere bei der Siedetemperatur des jeweiligen Lösungsmittels. Ein Zusatz einer anorganischen (z.B. NaH) oder organischen Base kann vorteilhaft sein. Besonders günstig ist auch das Arbeiten unter Phasentransferbedingungen, z.B. im System Dichlormethan/Wasser/NaOH/Katalysator, wobei sich als Katalysatoren insbesondere quartäre Ammoniumsalze eignen, z.B. Tetrabutylammoniumjodid.

Die Verbindungen der Formel I sind ferner durch Umsetzung von Verbindungen der Formel VIII mit Verbindungen der Formel IX erhältlich. Die Verbindungen der Formel VIII ($R^{17}$ = $A_2N$ bzw. AO) können aus den Verbindungen der Formel VI durch Reaktion mit Dialkylformamiddialkylacetalen bzw. Orthoameisensäuretrialkylestern erhalten werden, die Verbindungen der Formel VIII ($R^{17}$ = HO) daraus durch saure Hydrolyse. Die Verbindungen der Formel IX sind größtenteils bekannt.

Die Umsetzung von VIII mit IX gelingt im wesentlichen unter den gleichen Bedingungen wie diejenige von VI mit VII.

Es ist ferner möglich, in einer Verbindung der Formel I, die z. B. nach einer der oben angegebenen Reaktionen (a), (b), (c) oder (d) erhältlich ist, einen oder mehrere der Reste $R^1$ bis $R^6$ in andere Reste $R^1$ bis $R^6$ umzuwandeln.

Beispielsweise kann man eine Hydroxygruppe oder eine primäre oder sekundäre Aminogruppe alkylieren oder acylieren und/oder eine hydrolysierbare Gruppe, z. B. eine Ester-, CN-oder Säureamidgruppe hydrolysieren und/oder eine COOH-Gruppe verestern und/oder amidieren und/oder eine Verbindung, die sonst der Formel I entspricht, aber an Stelle eines oder mehrerer H-Atome eine oder mehrere reduzierbare Gruppen und/oder eine oder mehrere zusätzliche C-C-und/oder C-N-Bindungen enthält, mit einem reduzierenden Mittel behandeln und/oder ein H-Atom durch Halogenierung durch ein Hal-Atom ersetzen und/oder eine CN-Gruppe reduktiv in eine CHO-Gruppe umwandeln.

Eine Hydroxygruppe oder eine primäre oder sekundäre Aminogruppe kann durch Behandlung mit alkylierenden Mitteln in die entsprechende sekundäre oder tertiäre Aminogruppe umgewandelt werden. Der Ausdruck "alkylierende Mittel" wird hier in weitem Sinn verstanden und umfaßt z. B. auch Aralkylierende, AO-alkylierende, ArO-alkylierende, Ar-alkyl-O-alkylierende und $R^7R^8$N-alkylierende Mittel. Als alkylierende Mittel eignen sich z.B. Verbindungen der Formeln A-Hal, Ar-alkyl-Hal, AO-alkyl-Hal, ArO-alkyl-Hal, Ar-alkyl-O-alkyl-Hal oder $R^7R^8$N-alkyl-Hal (worin Hal Cl, Br oder J bedeutet) oder entsprechende Sulfonsäureester wie Methylchlorid, Methylbromid, Methyljodid, Dimethylsulfat, p-Toluolsulfonsäure-methylester, Ethylchlorid, Ethylbromid, Ethyljodid, Diethylsulfat, n-Propylchlorid, -bromid oder -jodid, Benzylchlorid, -bromid oder -jodid, 2-Methoxyethylchlorid, 2-Phenoxyethylbromid, 2-Benzyloxyethyljodid, 2-Morpholinoethylchlorid. Man kann ferner mit Aldehyden oder Ketonen unter Bildung von Aldehyd-Ammoniak-Verbindungen oder Schiffschen Basen kondensieren und diese anschließend entweder hydrieren oder mit einem Alkylierungsmittel behandeln und das erhaltene quartäre Salz anschließend hydrolysieren. Beispielsweise kann man ein primäres Amin durch Kondensation mit Benzaldehyd in die N-Benzylidenverbindung überführen und diese mit einem Alkylhalogenid in eines ihrer quartären Salze umwandeln, das nachfolgend z.B. durch Behandeln mit wässerigem Alkohol unter Abspaltung von Benzaldehyd in das sekundäre Amin übergeführt werden kann. Man kann ferner mit Aldehyden oder Ketonen unter reduzierenden Bedingungen alkylieren, z.B. in Gegenwart von Wasserstoff und einem Hydrierungskatalysator, oder in Gegenwart von $NaBH_4$ oder $NaCNBH_3$, wobei als Zwischenprodukte die entsprechenden Aldehyd-Ammoniake entstehen. Beispielsweise

kann man eine oder zwei Methylgruppen mit Formaldehyd in Gegenwart von Ameisensäure einführen. Weiterhin kann man mit einem Alkohol, der 1 -6 C-Atome besitzt, in Gegenwart von Raney-Nickel alkylieren. Die Alkylierung wird zweckmäßig in Gegenwart oder Abwesenheit eines der genannten inerten Lösungsmittel, z.B. DMF, bei Temperaturen zwischen etwa 0 und etwa 120 °, vorzugsweise zwischen 40 und 100 °, vorgenommen, wobei auch ein Katalysator zugegen sein kann, vorzugsweise eine Base wie Kalium-tert.-butylat oder NaH.

Als acylierende Mittel zur Acylierung von primären oder sekundären Aminen der Formel I eignen sich zweckmäßig die Halogenide (z.B. Chloride oder Bromide) oder Anhydride von Carbonsäuren der Formeln AcOH, z.B. Acetanhydrid, Propionylchlorid, Isobutyrylbromid, Ameisensäure-essigsäureanhydrid, Benzoylchlorid, p-Methoxybenzoylchlorid, Phenylacetylchlorid. Der Zusatz einer Base wie NaOH, KOH, Pyridin oder Triethylamin bei der Acylierung ist möglich, aber nicht notwendig. Die Acylierung erfolgt zweckmäßig in Gegenwart oder Abwesenheit eines inerten Lösungsmittels, z.B. eines Kohlenwasserstoffs wie Benzol oder Toluol, eines Nitrils wie Acetonitril, eines Amids wie DMF oder eines Überschusses einer tertiären Base wie Pyridin oder Triethylamin bei Temperaturen zwischen etwa 0 und etwa 160 °, vorzugsweise zwischen 20 und 120 °. Behandeln der Amine mit Isocyanaten führt analog zu den entsprechenden Harnstoffen (z. B. Verbindungen mit Ar = $R^{11}$NHCONH-phenyl).

In einer Verbindung der Formel I können, falls erwünscht, hydrolysierbare Gruppen hydrolysiert werden, zweckmäßig durch Behandeln mit Säuren, z.B. $H_2SO_4$, HCl, HBr, $H_3PO_4$, oder mit Basen, z.B. NaOH, KOH, $Na_2CO_3$ oder $K_2CO_3$, in wässeriger oder teilweise wässeriger Lösung, wobei ein zusätzliches inertes Lösungsmittel anwesend sein kann, z.B. ein Alkohol wie Methanol, Ethanol oder Isopropanol. Die Reaktionstemperaturen liegen zwischen etwa 0 und etwa 150 °, vorzugsweise zwischen 15 und 110 °.

Im einzelnen können z.B. Nitrilgruppen mit 50-80 %iger Schwefelsäure zu $CONH_2$-Gruppen oder unter kräftigeren Bedingungen, z.B. bei höheren Temperaturen, zu COOH-Gruppen hydrolysiert werden. Auch die Hydrolyse von $CONH_2$-zu COOH-Gruppen gelingt vorteilhaft mit 50 -80 %iger Schwefelsäure. Estergruppen können mit verdünnter wässerig-alkoholischer NaOH oder KOH verseift werden.

N-Acylgruppen können unter Bildung der freien Amine durch Behandeln mit wässerig-alkoholischen Basen, z.B. wässerig-methanolischer NaOH bei 60 -80 °, abgespalten werden. Analog können Phthalimidogruppen gespalten werden, schonender jedoch durch aufeinanderfolgendes Behandeln mit Hydrazinhydrat und alkoholischer Mineralsäure, z.B. methanolischer Salzsäure.

In einer Verbindung der Formel I enthaltene COOH-Gruppen können gewünschtenfalls verestert werden. Zur Veresterung eignen sich Alkohole der Formel A-OH und deren reaktionsfähige Derivate. Als reaktionsfähige Derivate kommen insbesondere die entsprechenden Metallalkoholate in Betracht, vorzugsweise die Alkalimetallalkoholate, z.B. Natrium-oder Kaliumalkoholate. Die Veresterung wird vorteilhaft in Gegenwart eines inerten Lösungsmittels durchgeführt. Gut geeignet sind insbesondere Ether wie Diethylether, Di-n-butylether, THF, Dioxan oder Anisol, Ketone wie Aceton, Butanon oder Cyclohexanon, Amide wie DMF oder Phosphorsäurehexamethyltriamid, Kohlenwasserstoffe wie Benzol, Toluol oder Xylol, Halogenkohlenwasserstoffe wie Tetrachlorkohlenstoff oder Tetrachlorethylen und Sulfoxide wie Dimethylsulfoxid oder Sulfolan. Mit Wasser nicht mischbare Lösungsmittel können gleichzeitig vorteilhaft zum azeotropen Abdestillieren des bei der Veresterung gebildeten Wassers verwendet werden. Gelegentlich kann auch ein Überschuß einer organischen Base, z.B. Pyridin, Chinolin oder Triethylamin als Lösungsmittel für die Veresterung angewandt werden. Die Veresterung kann auch in Abwesenheit eines Lösungsmittels, z.B. durch einfaches Erhitzen der Komponenten in Gegenwart von Natriumacetat, durchgeführt werden. Die Reaktionstemperatur liegt gewöhnlich zwischen -50 ° und +150 °, vorzugsweise zwischen -20 ° und +80 °. Bei diesen Temperaturen sind die Veresterungsreaktionen in der Regel nach 15 Minuten bis 48 Stunden beendet. Im einzelnen hängen die Reaktionsbedingungen für die Veresterung weitgehend von der Natur der verwendeten Ausgangsstoffe ab. So wird eine freie Carbonsäure mit einem freien Alkohol in der Regel in Gegenwart einer starken Säure, beispielsweise einer Mineralsäure wie Salzsäure oder Schwefelsäure, umgesetzt. Eine bevorzugte Reaktionsweise ist die Umsetzung eines Säureanhydrids oder insbesondere eines Säurechlorids mit einem Alkohol, vorzugsweise in einem basischen Milieu, wobei als Basen insbesondere Alkalimetallhydroxide wie Natrium-oder Kaliumhydroxid, Alkalimetallcarbonate bzw. -hydrogencarbonate wie Natriumcarbonat, Natriumhydrogencarbonat, Kaliumcarbonat oder Kaliumhydrogencarbonat, Alkalimetallacetate wie Natrium-oder Kaliumacetat, Erdalkalimetallhydroxide wie Calciumhydroxid oder organische Basen wie Triethylamin, Pyridin, Lutidin, Kollidin oder Chinolin von Bedeutung sind. Besonders - schonende Veresterungen gelingen mit Hilfe der bei der Peptidsynthese gebräuchlichen Dehydratisierungsmitteln wie Dicyclohexylcarbodiimid.

Weiterhin kann man Carbonsäuren mit anorganischen Säurechloriden oder -bromiden wie $SOCl_2$ oder $PBr_3$ in die entsprechenden Säurehalogenide umwandeln, zweckmäßig in Gegenwart eines inerten Lösungsmittels wie DMF bei 0 -30°. Aus diesen können durch Reaktion mit Ammoniak oder Aminen die entsprechenden Säureamide erhalten werden, zweckmäßig in Gegenwart eines inerten Lösungsmittels wie DMF bei 0 -30°.

Man kann ferner reduzierbare Gruppen, z. B. Nitrogruppen zu Aminogruppen, reduzieren und/oder Mehrfachbindungen, z. B. C-C-Doppelbindungen, hydrieren. Eine Reduktion erfolgt z. B. durch katalytische Hydrierung oder auf chemischem Wege. Fur katalytische Hydrierungen eignen sich beispielsweise Edelmetall-, Nickel-oder Kobaltkatalysatoren. Als Edelmetalle kommen in erster Linie Platin oder Palladium in Betracht, die auf Trägern wie Kohle oder Calciumcarbonat, als Oxide wie Platinoxid oder in feinteiliger Form vorliegen können. Bevorzugte Katalysatoren sind Pd-Kohle und Raney-Nickel. Man hydriert zweckmäßig bei Drucken zwischen 1 und 200, vorzugsweise 1 und 10 bar und bei Temperaturen zwischen 0 und 150°, vorzugsweise 15 und 60°, in Gegenwart eines der angegebenen inerten Lösungsmittel, vorzugsweise Methanol, Ethanol, Isopropanol oder Essigsäure.

Weiterhin kann man H-Atome durch Hal-Atome ersetzen, indem man sie mit halogenierenden Mitteln behandelt. So kann man z. B. in Verbindungen der Formel I, in denen $R^2$ $CH_3$ bedeutet, mit Hilfe von Pyridiniumbromidperbromid in einem inerten Lösungsmittel wie Dichlormethan bei Temperaturen zwischen etwa -10 und + 30°, vorzugsweise 0 und 10°, ein Bromatom in die Methylgruppe einführen. 2-Brommethyl-3-alkoxycarbonyl-1,4-dihydropyridine der Formel I, die z. B. auf diesem Wege erhältlich sind, können leicht zu den entsprechenden Lactonen I ($R^2$ und $R^3$ zusammen = $-CH_2-O-CO-$) hydrolysiert werden, z. B. durch etwa 0,5 -2 std. Erhitzen mit feuchten inerten Lösungsmitteln wie Acetonitril; als Neben-und Zwischenprodukt bildet sich das 2-Hydroxymethyl-3-alkoxycarbonyl-1,4-dihydropyridin.

Ferner kann man CN-Gruppen reduktiv in CHO-Gruppen umwandeln, besonders vorteilhaft, indem man die Nitrile mit Raney-Nickel in einem inerten Lösungsmittel oder Lösungsmittelgemisch, z. B. Pyridin/Wasser/Essigsäure bei Temperaturen zwischen etwa 0 und etwa 60°, vorzugsweise 15 und 30°, behandelt.

Eine Base der Formel I kann mit einer Säure in das zugehörige Säureadditionssalz übergeführt werden. Für diese Umsetzung kommen Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein-oder mehrbasige Carbon-, Sulfon-oder Schwefelsäuren, z.B. Ameisensäure, Essigsäure, Propionsäure, Pivalinsäure, Diethylessig säure, Malonsäure, Bernsteinsäure, Pimelinsäure, Fumarsäure, Maleinsäure, Milchsäure, Weinsäure, Äpfelsäure, Benzoesäure, Salicylsäure, 2-oder 3-Phenylpropionsäure, Citronensäure, Gluconsäure, Ascorbinsäure, Nicotinsäure, Isonicotinsäure, Methan-oder Ethansulfonsäure, Ethandisulfonsäure, 2-Hydroxyethansulfonsäure, Benzolsulfonsäure, p-Toluolsulfonsäure, Naphthalin-mono-und -disulfonsäuren, Laurylschwefelsäure. Salze mit physiologisch nicht unbedenklichen Säuren, z. B. Pikrate, können zur Isolierung oder Aufreinigung der Verbindungen der Formel I verwendet werden.

Die freien Basen der Formel I können, falls gewünscht, aus ihren Salzen durch Behandlung mit starken Basen wie Natrium-oder Kaliumhydroxid, Natrium-oder Kaliumcarbonat in Freiheit gesetzt werden.

Andererseits können Verbindungen der Formel I mit freien COOH-Gruppen durch Umsetzung mit Basen in ihre Metall-bzw. Ammoniumsalze umgewandelt werden. Als Salze kommen insbesondere die Natrium-, Kalium-, Magnesium-, Calcium-und Ammoniumsalze in Betracht, ferner substituierte Ammoniumsalze, z. B. die Dimethyl-, Diethyl-, Monoethanol-, Diethanol-, Triethanol-, Cyclohexyl-oder Dicyclohexylammoniumsalze.

Die Verbindungen der Formel I können ein oder mehrere Asymmetriezentren enthalten. In diesem Fall liegen sie gewöhnlich in racemischer Form vor. Erhaltene Racemate können nach an sich bekannten Methoden mechanisch oder chemisch in ihre optischen Antipoden getrennt werden. Vorzugsweise werden aus dem racemischen Gemisch durch Umsetzung mit einem optisch aktiven Trennmittel Diastereomere gebildet. Als Trennmittel eignen sich z.B. optisch aktive Säuren, wie die D-und L-Formen von Wein säure, Diacetylweinsäure, Dibenzoylweinsäure, Mandelsäure, Äpfelsäure, Milchsäure oder die verschiedenen optisch aktiven Camphersulfonsäuren wie ß-Camphersulfonsäure.

Natürlich ist es auch möglich, optisch aktive Verbindungen der Formel I nach den oben beschriebenen Methoden zu erhalten, indem man Ausgangsstoffe verwendet, die bereits optisch aktiv sind.

Die enantiomeren Formen optisch aktiver Verbindungen der Formel I können sich in ihren pharmakologischen Eigenschaften wie z. B. in der calciumantagonistischen bzw. -agonistischen Wirkung graduell oder grundsätzlich unterscheiden, sie können aber auch gleich sein.

Gegenstand der Erfindung ist ferner die Verwendung der Verbindungen der Formel I und ihrer physiologisch unbedenklichen Salze zur Herstellung pharmazeutischer Zubereitungen, insbesondere auf nicht-chemischen Wege. Hierbei können sie zusammen mit mindestens einem festen, flüssigen und/oder halbflüssigen Träger-oder Hilfsstoff und gegebenenfalls in Kombination mit einem oder mehreren weiteren Wirkstoffen in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Salze.

Diese Zubereitungen können als Arzneimittel in der Human-oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Alkylenglykole, Polyethylenglykole, Glycerintriacetat, Gelatine, Kohlehydrate wie Lactose oder Stärke, Magnesiumstearat, Talk, Vaseline.

Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wäßrige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden.

Die angegebenen Zubereitungen können sterilisiert sein und/oder Hilfsstoffe wie Gleit-, Konservierungs-, Stabilisierungs-und/oder Netzmittel, Emulgatoren, Salze zur Beeinflussung des osmotischen Druckes, Puffersubstanzen, Farb-, Geschmacks-und/oder Aromastoffe enthalten. Sie können, falls erwünscht, auch einen oder mehrere weitere Wirkstoffe enthalten, z.B. ein oder mehrere Vitamine.

Gegenstand der Erfindung ist ferner die Verwendung der Verbindungen der Formel I bei der Bekampfung von Krankheiten, insbesondere der Herzinsuffizienz, der arteriellen Hypertonie, der Koronarsklerose, der Atherosklerose, der Arrhythmie und insbesondere der Angina pectoris, ferner von Krankheiten, die mit Hypertonie verbunden sind, von pulmonalem Hochdruck, dem Raynaudschen Phänomen, Migräne, Asthma, Herzhypertrophie, Ischämie, Myokardinfarkt, weiterhin von Dauerspasmen innerer Organe (Ösophagusspasmen, Achalasie), Ureterspasmen, Harninkontinenz, irri tabler Blase, frühzeitigen Wehen, Dysmenorrhöe, Darmspasmen, zerebrovaskulären Durchblutungsstörungen, Vertigo, Seekrankheit, Epilepsie, vertebrobasilärer Insuffizienz, endokrinologischen Störungen.

Dabei werden die erfindungsgemäßen Substanzen in der Regel in Analogie zu bekannten Calciumantagonisten, wie Verapamil oder Nifedipin, an Menschen oder Säugetiere wie Affen, Hunde, Katzen, Ratten oder Mäuse verabreicht, an Menschen vorzugsweise in Dosierungen zwischen etwa 5 und 500 mg, insbesondere zwischen 20 und 100 mg pro Dosierungseinheit. Die tägliche Dosierung liegt vorzugsweise zwischen etwa 0,1 und 10, insbesondere zwischen 0,4 und 2 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Die folgenden Beispiele sollen die Erfindung erläutern ohne sie zu begrenzen. Alle Temperaturen sind in Grad Celsius angegeben. "Übliche Aufarbeitung" bedeutet: man setzt, falls erforderlich, Wasser und Dichlormethan oder Ethylacetat hinzu, schüttelt, trennt ab, wäscht die organische Phase mit Wasser, trocknet über $Na_2SO_4$, dampft ein und reinigt den Rückstand durch Chromatographie an Kieselgel und/oder Kristallisation.

Beispiel 1

Eine Lösung von 2,65 g 2-(o-Formylphenyl)-4-phenylthiazol [F. 99-100 °; erhältlich durch Reaktion von o-Dimethoxymethyl-benzonitril mit $H_2S$ zu o-Dimethoxymethyl-thiobenzamid (F. 100-102 °), Umsetzung mit α-Bromacetophenon zu o-Dimethoxymethyl-thiobenzoesäureimid-S-phenacylester (Öl), Cyclisierung zu 2-(o-Dimethoxymethylphenyl)-4-phenylthiazol durch Kochen mit Methanol und anschließende Hydrolyse mit wässeriger Salzsäure], 1,06 ml 33 %ig. wäßrigem Ammoniak, 2,32 g Acetessigsäuremethylester und 120 ml Methanol wird 18 Std. gekocht und eingedampft. Nach Behandeln des Rückstandes mit Ether erhält man 2,6-Dimethyl-3,5-dimethoxycarbonyl-4-o-(4-phenyl-2-thiazolyl)-phenyl-1,4-dihydropyridin, F. 145-148 ° - (Zers.).

Analog erhält man mit den entsprechenden Aldehyden die folgenden 2,6-Dimethyl-3,5-dimethoxycarbonyl-1,4-dihydropyridine:

4-o-(2-Thiazolyl)-phenyl-
4-m-(2-Thiazolyl)-phenyl-
4-o-(4-Methyl-2-thiazolyl)-phenyl-
4-m-(4-Methyl-2-thiazolyl)-phenyl-
4-o-(4,5-Dimethyl-2-thiazolyl)-phenyl-
4-m-(4,5-Dimethyl-2-thiazolyl)-phenyl-
4-o-(4-Methyl-5-phenyl-2-thiazolyl)-phenyl-
4-m-(4-Methyl-5-phenyl-2-thiazolyl)-phenyl-
4-o-(4-Ethoxycarbonyl-2-thiazolyl)-phenyl-
4-m-(4-Ethoxycarbonyl-2-thiazolyl)-phenyl-
4-o-(4-Ethoxycarbonyl-5-benzyl-2-thiazolyl)-phenyl-
4-m-(4-Ethoxycarbonyl-5-benzyl-2-thiazolyl)-phenyl-
4-o-(4-Ethoxycarbonylmethyl-2-thiazolyl)-phenyl-
4-m-(4-Ethoxycarbonylmethyl-2-thiazolyl)-phenyl-
4-o-[4-(2-Pyridyl)-2-thiazolyl]-phenyl-
4-m-[4-(2-Pyridyl)-2-thiazolyl]-phenyl-
4-o-[4-(3-Pyridyl)-2-thiazolyl]-phenyl-
4-m-[4-(3-Pyridyl)-2-thiazolyl]-phenyl-
4-o-[4-(4-Pyridyl)-2-thiazolyl]-phenyl-
4-m-[4-(4-Pyridyl)-2-thiazolyl]-phenyl-
4-o-[4-(3-Cyan-6-methyl-2-pyridon-5-yl)-2-thiazolyl]-phenyl-
4-m-[4-(3-Cyan-6-methyl-2-pyridon-5-yl)-2-thiazolyl]-phenyl-
4-m-(4-Phenyl-2-thiazolyl)-phenyl-
4-o-(4-Phenyl-5-methyl-2-thiazolyl)-phenyl-
4-m-(4-Phenyl-5-methyl-2-thiazolyl)-phenyl-
4-o-(4-o-Tolyl-2-thiazolyl)-phenyl-
4-m-(4-o-Tolyl-2-thiazolyl)-phenyl-
4-o-(4-m-Tolyl-2-thiazolyl)-phenyl-, F. 170-172 °
4-m-(4-m-Tolyl-2-thiazolyl)-phenyl-
4-o-(4-p-Tolyl-2-thiazolyl)-phenyl-, F. 124-127 ° (Zers.)
4-m-(4-p-Tolyl-2-thiazolyl)-phenyl-
4-o-[4-(2,4,6-Trimethylphenyl)-2-thiazolyl]-phenyl-
4-m-[4-(2,4,6-Trimethylphenyl)-2-thiazolyl]-phenyl-
4-o-[4-(2,6-Dimethyl-4-tert.-butylphenyl)-2-thiazolyl]-phenyl-
4-m-[4-(2,6-Dimethyl-4-tert.-butylphenyl)-2-thiazolyl]-phenyl-
4-o-(4-o-Methoxyphenyl-2-thiazolyl)-phenyl-
4-m-(4-o-Methoxyphenyl-2-thiazolyl)-phenyl-
4-o-(4-m-Methoxyphenyl-2-thiazolyl)-phenyl-
4-m-(4-m-Methoxyphenyl-2-thiazolyl)-phenyl-
4-o-(4-p-Methoxyphenyl-2-thiazolyl)-phenyl-, F. 110-114 ° (Zers.)
4-m-(4-p-Methoxyphenyl-2-thiazolyl)-phenyl-
4-o-[4-(2,5-Dimethoxyphenyl)-2-thiazolyl]-phenyl-
4-m-[4-(2,5-Dimethoxyphenyl)-2-thiazolyl]-phenyl-
4-o-[4-(3,4-Dimethoxyphenyl)-2-thiazolyl]-phenyl-
4-m-[4-(3,4-Dimethoxyphenyl)-2-thiazolyl]-phenyl-
4-o-[4-(3,4-Dimethoxyphenyl)-5-methyl-2-thiazolyl]-phenyl-
4-m-[4-(3,4-Dimethoxyphenyl)-5-methyl-2-thiazolyl]-phenyl-
4-o-[4-(3,4,5-Trimethoxyphenyl)-2-thiazolyl]-phenyl-
4-m-[4-(3,4,5-Trimethoxyphenyl)-2-thiazolyl]-phenyl-
4-o-(4-p-Ethoxyphenyl-2-thiazolyl)-phenyl-
4-m-(4-p-Ethoxyphenyl-2-thiazolyl)-phenyl-
4-o-(4-o-Fluorphenyl-2-thiazolyl)-phenyl-
4-m-(4-o-Fluorphenyl-2-thiazolyl)-phenyl-
4-o-(4-m-Fluorphenyl-2-thiazolyl)-phenyl-
4-m-(4-m-Fluorphenyl-2-thiazolyl)-phenyl-
4-o-(4-p-Fluorphenyl-2-thiazolyl)-phenyl-

4-m-(4-p-Fluorphenyl-2-thiazolyl)-phenyl-
4-o-(4-o-Chlorphenyl-2-thiazolyl)-phenyl-
4-m-(4-o-Chlorphenyl-2-thiazolyl)-phenyl-
4-o-(4-m-Chlorphenyl-2-thiazolyl)-phenyl-
4-m-(4-m-Chlorphenyl-2-thiazolyl)-phenyl-
4-o-(4-p-Chlorphenyl-2-thiazolyl)-phenyl-, F. 206-208 ° (Zers.)
4-m-(4-p-Chlorphenyl-2-thiazolyl)-phenyl-
4-o-(4-p-Chlorphenyl-5-methyl-2-thiazolyl)-phenyl-
4-m-(4-p-Chlorphenyl-5-methyl-2-thiazolyl)-phenyl-
4-o-]4-(2,4-Dichlorphenyl)-2-thiazolyl]-phenyl-
4-m-]4-(2,4-Dichlorphenyl)-2-thiazolyl]-phenyl-
4-o-[4-(3,4-Dichlorphenyl)-2-thiazolyl]-phenyl-, F. 108-110 °
4-m-[4-(3,4-Dichlorphenyl)-2-thiazolyl]-phenyl-
4-o-(4-p-Bromphenyl-2-thiazolyl)-phenyl-
4-m-(4-p-Bromphenyl-2-thiazolyl)-phenyl-
4-o-[4-(2,4-Dimethoxy-5-bromphenyl)-2-thiazolyl]-phenyl-
4-m-[4-(2,4-Dimethoxy-5-bromphenyl)-2-thiazolyl]-phenyl-
4-o-(4-m-Trifluormethylphenyl-2-thiazolyl)-phenyl-
4-m-(4-m-Trifluormethylphenyl-2-thiazolyl)-phenyl-
4-o-(4-o-Hydroxyphenyl-2-thiazolyl)-phenyl-
4-m-(4-o-Hydroxyphenyl-2-thiazolyl)-phenyl-
4-o-(4-m-Hydroxyphenyl-2-thiazolyl)-phenyl-
4-m-(4-m-Hydroxyphenyl-2-thiazolyl)-phenyl-
4-o-(4-p-Hydroxyphenyl-2-thiazolyl)-phenyl-
4-m-(4-p-Hydroxyphenyl-2-thiazolyl)-phenyl-
4-o-[4-(3,4-Dihydroxyphenyl)-2-thiazolyl]-phenyl-
4-m-[4-(3,4-Dihydroxyphenyl)-2-thiazolyl]-phenyl-
4-o-(4-o-Nitrophenyl-2-thiazolyl)-phenyl-
4-m-(4-o-Nitrophenyl-2-thiazolyl)-phenyl-
4-o-(4-m-Nitrophenyl-2-thiazolyl)-phenyl-
4-m-(4-m-Nitrophenyl-2-thiazolyl)-phenyl-
4-o-(4-p-Nitrophenyl-2-thiazolyl)-phenyl-
4-m-(4-p-Nitrophenyl-2-thiazolyl)-phenyl-
4-o-[4-(3-Nitro-4-methoxyphenyl)-2-thiazolyl]-phenyl-
4-m-[4-(3-Nitro-4-methoxyphenyl)-2-thiazolyl]-phenyl-
4-o-(4-p-Dimethylaminophenyl-2-thiazolyl)-phenyl-
4-m-(4-p-Dimethylaminophenyl-2-thiazolyl)-phenyl-
4-o-(4-p-Acetamidophenyl-2-thiazolyl)-phenyl-
4-m-(4-p-Acetamidophenyl-2-thiazolyl)-phenyl-
4-o-[4-(3-Acetamido-4-methoxyphenyl)-2-thiazolyl]-phenyl-
4-m-[4-(3-Acetamido-4-methoxyphenyl)-2-thiazolyl]-phenyl-
4-o-(4-p-Cyanphenyl-2-thiazolyl)-phenyl-
4-m-(4-p-Cyanphenyl-2-thiazolyl)-phenyl-
4-o-(4-p-Carbamoylphenyl-2-thiazolyl)-phenyl-
4-m-(4-p-Carbamoylphenyl-2-thiazolyl)-phenyl-
4-o-(4-o-Sulfamoylphenyl-2-thiazolyl)-phenyl-
4-m-(4-o-Sulfamoylphenyl-2-thiazolyl)-phenyl-
4-o-(4-m-Sulfamoylphenyl-2-thiazolyl)-phenyl-
4-m-(4-m-Sulfamoylphenyl-2-thiazolyl)-phenyl-
4-o-(4-p-Sulfamoylphenyl-2-thiazolyl)-phenyl-
4-m-(4-p-Sulfamoylphenyl-2-thiazolyl)-phenyl-
4-o-(4-p-Difluormethoxyphenyl-2-thiazolyl)-phenyl-
4-m-(4-p-Difluormethoxyphenyl-2-thiazolyl)-phenyl-
4-o-[4-(4-Biphenylyl)-2-thiazolyl]-phenyl-

4-m-[4-(4-Biphenylyl)-2-thiazolyl]-phenyl-
4-o-(4-p-Phenoxyphenyl-2-thiazolyl)-phenyl-
4-m-(4-p-Phenoxyphenyl-2-thiazolyl)-phenyl-.

Beispiel 2

Analog Beispiel 1 erhält man mit Acetessigsäureethylester die folgenden 2,6-Dimethyl-3,5-diethoxycarbonyl-1,4-dihydropyridine:
4-o-(2-Thiazolyl)-phenyl-
4-m-(2-Thiazolyl)-phenyl-
4-o-(4-Methyl-2-thiazolyl)-phenyl-
4-m-(4-Methyl-2-thiazolyl)-phenyl-
4-o-(4,5-Dimethyl-2-thiazolyl)-phenyl-
4-m-(4,5-Dimethyl-2-thiazolyl)-phenyl-
4-o-(4-Methyl-5-phenyl-2-thiazolyl)-phenyl-
4-m-(4-Methyl-5-phenyl-2-thiazolyl)-phenyl-
4-o-(4-Ethoxycarbonyl-2-thiazolyl)-phenyl-
4-m-(4-Ethoxycarbonyl-2-thiazolyl)-phenyl-
4-o-(4-Ethoxycarbonyl-5-benzyl-2-thiazolyl)-phenyl-
4-m-(4-Ethoxycarbonyl-5-benzyl-2-thiazolyl)-phenyl-
4-o-(4-Ethoxycarbonylmethyl-2-thiazolyl)-phenyl-
4-m-(4-Ethoxycarbonylmethyl-2-thiazolyl)-phenyl-
4-o-[4-(2-Pyridyl)-2-thiazolyl]-phenyl-
4-m-[4-(2-Pyridyl)-2-thiazolyl]-phenyl-
4-o-[4-(3-Pyridyl)-2-thiazolyl]-phenyl-
4-m-[4-(3-Pyridyl)-2-thiazolyl]-phenyl-
4-o-[4-(4-Pyridyl)-2-thiazolyl]-phenyl-
4-m-[4-(4-Pyridyl)-2-thiazolyl]-phenyl-
4-o-[4-(3-Cyan-6-methyl-2-pyridon-5-yl)-2-thiazolyl]-phenyl-
4-m-[4-(3-Cyan-6-methyl-2-pyridon-5-yl)-2-thiazolyl]-phenyl-
4-o-(4-Phenyl-2-thiazolyl)-phenyl-
4-m-(4-Phenyl-2-thiazolyl)-phenyl-
4-o-(4-Phenyl-5-methyl-2-thiazolyl)-phenyl-
4-m-(4-Phenyl-5-methyl-2-thiazolyl)-phenyl-
4-o-(4-o-Tolyl-2-thiazolyl)-phenyl-
4-m-(4-o-Tolyl-2-thiazolyl)-phenyl-
4-o-(4-m-Tolyl-2-thiazolyl)-phenyl-, F. 144-146 °
4-m-(4-m-Tolyl-2-thiazolyl)-phenyl-
4-o-(4-p-Tolyl-2-thiazolyl)-phenyl-
4-m-(4-p-Tolyl-2-thiazolyl)-phenyl-
4-o-[4-(2,4,6-Trimethylphenyl)-2-thiazolyl]-phenyl-
4-m-[4-(2,4,6-Trimethylphenyl)-2-thiazolyl]-phenyl-
4-o-[4-(2,6-Dimethyl-4-tert.-butylphenyl)-2-thiazolyl]-phenyl-
4-m-[4-(2,6-Dimethyl-4-tert.-butylphenyl)-2-thiazolyl]-phenyl-
4-o-(4-o-Methoxyphenyl-2-thiazolyl)-phenyl-
4-m-(4-o-Methoxyphenyl-2-thiazolyl)-phenyl-
4-o-(4-m-Methoxyphenyl-2-thiazolyl)-phenyl-
4-m-(4-m-Methoxyphenyl-2-thiazolyl)-phenyl-
4-o-(4-p-Methoxyphenyl-2-thiazolyl)-phenyl-, F. 184-186 °
4-m-(4-p-Methoxyphenyl-2-thiazolyl)-phenyl-
4-o-[4-(2,5-Dimethoxyphenyl)-2-thiazolyl]-phenyl-
4-m-[4-(2,5-Dimethoxyphenyl)-2-thiazolyl]-phenyl-
4-o-[4-(3,4-Dimethoxyphenyl)-2-thiazolyl]-phenyl-
4-m-[4-(3,4-Dimethoxyphenyl)-2-thiazolyl]-phenyl-
4-o-[4-(3,4-Dimethoxyphenyl)-5-methyl-2-thiazolyl]-phenyl-
4-m-[4-(3,4-Dimethoxyphenyl)-5-methyl-2-thiazolyl]-phenyl-
4-o-[4-(3,4,5-Trimethoxyphenyl)-2-thiazolyl]-phenyl-

4-m-[4-(3,4,5-Trimethoxyphenyl)-2-thiazolyl]-phenyl-
4-o-(4-p-Ethoxyphenyl-2-thiazolyl)-phenyl-
4-m-(4-p-Ethoxyphenyl-2-thiazolyl)-phenyl-
4-o-(4-o-Fluorphenyl-2-thiazolyl)-phenyl-
4-m-(4-o-Fluorphenyl-2-thiazolyl)-phenyl-
4-o-(4-m-Fluorphenyl-2-thiazolyl)-phenyl-
4-m-(4-m-Fluorphenyl-2-thiazolyl)-phenyl-
4-o-(4-p-Fluorphenyl-2-thiazolyl)-phenyl-
4-m-(4-p-Fluorphenyl-2-thiazolyl)-phenyl-
4-o-(4-o-Chlorphenyl-2-thiazolyl)-phenyl-
4-m-(4-o-Chlorphenyl-2-thiazolyl)-phenyl-
4-o-(4-m-Chlorphenyl-2-thiazolyl)-phenyl-
4-m-(4-m-Chlorphenyl-2-thiazolyl)-phenyl-
4-o-(4-p-Chlorphenyl-2-thiazolyl)-phenyl-
4-m-(4-p-Chlorphenyl-2-thiazolyl)-phenyl-
4-o-(4-p-Chlorphenyl-5-methyl-2-thiazolyl)-phenyl-
4-m-(4-p-Chlorphenyl-5-methyl-2-thiazolyl)-phenyl-
4-o-[4-(2,4-Dichlorphenyl)-2-thiazolyl]-phenyl-
4-m-[4-(2,4-Dichlorphenyl)-2-thiazolyl]-phenyl-
4-o-[4-(3,4-Dichlorphenyl)-2-thiazolyl]-phenyl-, F. 228-232 ° (Zers.)
4-m-[4-(3,4-Dichlorphenyl)-2-thiazolyl]-phenyl-
4-o-(4-p-Bromphenyl-2-thiazolyl)-phenyl-
4-m-(4-p-Bromphenyl-2-thiazolyl)-phenyl-
4-o-[4-(2,4-Dimethoxy-5-bromphenyl)-2-thiazolyl]-phenyl-
4-m-[4-(2,4-Dimethoxy-5-bromphenyl)-2-thiazolyl]-phenyl-
4-o-(4-m-Trifluormethylphenyl-2-thiazolyl)-phenyl-
4-m-(4-m-Trifluormethylphenyl-2-thiazolyl)-phenyl-
4-o-(4-o-Hydroxyphenyl-2-thiazolyl)-phenyl-
4-m-(4-o-Hydroxyphenyl-2-thiazolyl)-phenyl-
4-o-(4-m-Hydroxyphenyl-2-thiazolyl)-phenyl-
4-m-(4-m-Hydroxyphenyl-2-thiazolyl)-phenyl-
4-o-(4-p-Hydroxyphenyl-2-thiazolyl)-phenyl-
4-m-(4-p-Hydroxyphenyl-2-thiazolyl)-phenyl-
4-o-[4-(3,4-Dihydroxyphenyl)-2-thiazolyl]-phenyl-
4-m-[4-(3,4-Dihydroxyphenyl)-2-thiazolyl]-phenyl-
4-o-(4-o-Nitrophenyl-2-thiazolyl)-phenyl-
4-m-(4-o-Nitrophenyl-2-thiazolyl)-phenyl-
4-o-(4-m-Nitrophenyl-2-thiazolyl)-phenyl-
4-m-(4-m-Nitrophenyl-2-thiazolyl)-phenyl-
4-o-(4-p-Nitrophenyl-2-thiazolyl)-phenyl-
4-m-(4-p-Nitrophenyl-2-thiazolyl)-phenyl-
4-o-[4-(3-Nitro-4-methoxyphenyl)-2-thiazolyl]-phenyl-
4-m-[4-(3-Nitro-4-methoxyphenyl)-2-thiazolyl]-phenyl-
4-o-(4-p-Dimethylaminophenyl-2-thiazolyl)-phenyl-
4-m-(4-p-Dimethylaminophenyl-2-thiazolyl)-phenyl-
4-o-(4-p-Acetamidophenyl-2-thiazolyl)-phenyl-
4-m-(4-p-Acetamidophenyl-2-thiazolyl)-phenyl-
4-o-[4-(3-Acetamido-4-methoxyphenyl)-2-thiazolyl]-phenyl-
4-m-[4-(3-Acetamido-4-methoxyphenyl)-2-thiazolyl]-phenyl-
4-o-(4-p-Cyanphenyl-2-thiazolyl)-phenyl-
4-m-(4-p-Cyanphenyl-2-thiazolyl)-phenyl-
4-o-(4-p-Carbamoylphenyl-2-thiazolyl)-phenyl-
4-m-(4-p-Carbamoylphenyl-2-thiazolyl)-phenyl-
4-o-(4-o-Sulfamoylphenyl-2-thiazolyl)-phenyl-
4-m-(4-o-Sulfamoylphenyl-2-thiazolyl)-phenyl-
4-o-(4-m-Sulfamoylphenyl-2-thiazolyl)-phenyl-
4-m-(4-m-Sulfamoylphenyl-2-thiazolyl)-phenyl-
4-o-(4-p-Sulfamoylphenyl-2-thiazolyl)-phenyl-

4-m-(4-p-Sulfamoylphenyl-2-thiazolyl)-phenyl-
4-o-(4-p-Difluormethoxyphenyl-2-thiazolyl)-phenyl-
4-m-(4-p-Difluormethoxyphenyl-2-thiazolyl)-phenyl-
4-o-[4-(4-Biphenylyl)-2-thiazolyl]-phenyl-
4-m-[4-(4-Biphenylyl)-2-thiazolyl]-phenyl-
4-o-(4-p-Phenoxyphenyl-2-thiazolyl)-phenyl-
4-m-(4-p-Phenoxyphenyl-2-thiazolyl)-phenyl-.

Beispiel 3

Man erhitzt eine Lösung von 4,07 g 2-Acetyl-o-(4-p-methoxyphenyl-2-thiazolyl)-zimtsäure-ethylester [cis-trans-Gemisch; erhältlich aus 2-o-Formylphenyl-4-p-methoxyphenyl-thiazol und Acetessigsäureethylester], 0,7 ml 33 %ig. wäßrigem Ammoniak und 1,3 g Acetessigsäureethylester in 40 ml Methanol 24 Std. zum Sieden, arbeitet analog Beispiel 1 auf und erhält 2,6-Dimethyl-3,5-diethoxycarbonyl-4-o-(4-p-methoxyphenyl-2-thiazolyl)-phenyl-1,4-dihydropyridin, F. 184-186 °.

Analog erhält man mit den entsprechenden Ketoestern die nachstehenden 2,6-Dimethyl-4-o-(4-p-methoxyphenyl-2-thiazolyl)-phenyl-1,4-dihydropyridine:
-3-methoxycarbonyl-5-ethoxycarbonyl-
-3-methoxycarbonyl-5-isopropoxycarbonyl-
-3-methoxycarbonyl-5-isobutoxycarbonyl-
-3-methoxycarbonyl-5-(2-N-benzyl-N-methylamino-ethoxycarbonyl)-
-3-propoxycarbonyl-5-(2-propoxyethoxycarbonyl)-
-3-isopropoxycarbonyl-5-(2-methoxyethoxycarbonyl)-.

Beispiel 4

(a) Analog Beispiel 3 erhält man aus 2-Acetyl-o-(4-phenyl-2-thiazolyl)-zimtsäureethylester, 4-Acetoxy-3-oxobutansäureethylester und Ammoniak das 2-Acetoxymethyl-3,5-diethoxycarbonyl-4-o-(4-phenyl-2-thiazolyl)-phenyl-6-methyl-1,4-dihydropyridin, Öl.

(b) Man kocht 1 g der nach (a) erhaltenen Verbindung 1 Std. mit 15 ml mit HCl gesättigtem Dioxan, dampft ein, chromatographiert und erhält 2-Methyl-3-ethoxycarbonyl-4-o-(4-phenyl-2-thiazolyl)-phenyl-5-oxo-1,4,5,7-tetrahydrofuro[3,4-b]pyridin.

Beispiel 5

Analog Beispiel 3 erhält man aus 2-Acetyl-o-(4-phenyl-2-thiazolyl)-zimtsäureethylester, Tetronsäure - (oder Tetronsäuremethylester) und Ammoniak das 2-Methyl-3-ethoxycarbonyl-4-o-(4 phenyl-2-thiazolyl)-phenyl-5-oxo-1,4,5,7-tetrahydrofuro[3,4-b]pyridin.

Beispiel 6

(a) Analog Beispiel 3 erhält man aus 2-Acetyl-o-(4-phenyl-2-thiazolyl)-zimtsäureethylester, 3-Oxo-4,4-ethylendioxybuttersäureethylester und Ammoniak das 2-Methyl-3,5-diethoxycarbonyl-4-o-(4-phenyl-2-thiazolyl)-phenyl-6-ethylendioxymethyl-1,4-dihydropyridin.

(b) Das nach (a) erhaltene Produkt (1 g) wird mit 50 ml 10 %iger methanolischer Salzsäure gekocht - (dünnschichtchromatographische Kontrolle). Man erhält 2-Methyl-3,5-diethoxycarbonyl-4-o-(4-phenyl-2-thiazolyl)-phenyl-6-formyl-1,4-dihydropyridin.

Beispiel 7

Man erhitzt eine Lösung von 4,07 g 2-Acetyl-o-(4-p-methoxyphenyl-2-thiazolyl)-zimtsäureethylester und 1,29 g 3-Methylaminocrotonsäuremethylester in 50 ml Ethanol 16 Std. zum Sieden, dampft ein, chromatographiert den Rückstand an Kieselgel und erhält 1,2,6-Trimethyl-3-methoxycarbonyl-4-o-(4-p-methoxyphenyl-2-thiazolyl)-phenyl-5-ethoxycarbonyl-1,4-dihydropyridin.

Analog erhält man mit 3-Aminocrotonsäurenitril 2,6-Dimethyl-3-ethoxycarbonyl-4-o-(4-p-methoxyphenyl-2-thiazolyl)-phenyl-5-cyan-1,4-dihydropyridin.

Beispiel 8

Ein Gemisch von 4,07 g 2-Acetyl-o-(4-p-methoxyphenyl-2-thiazolyl)-zimtsäureethylester und 1 g Tetronsäureamid (vgl. DE-OS 3311003) in 80 ml Methanol wird 24 Std. gekocht und eingedampft. Nach Chromatographie des Rückstandes an Kieselgel erhält man 2-Methyl-3-ethoxycarbonyl-4-o-(4-p-methoxyphenyl-2-thiazolyl)-phenyl-5-oxo-1,4,5,7-tetrahydrofuro[3,4-b]-pyridin.

An Stelle des Tetronsäureamids kann man auch 1,87 g 3-Amino-4-acetoxy-crotonsäureethylester verwenden.

Das gleiche Produkt ist analog erhältlich aus äquimolaren Mengen von

(a) 2-o-Formylphenyl-4-p-methoxyphenyl-thiazol, 4-Chlor-3-oxobuttersäureethylester und 3-Aminocrotonsäureethylester,

(b) 2-o-Formylphenyl-4-p-methoxyphenyl-thiazol, 4-Acetoxy-3-aminocrotonsäureethylester und Acetessigsäureethylester, wobei jedoch das zunächst erhaltene rohe 2-Methyl-3,5-diethoxycarbonyl-4-o-(4-p-methoxyphenyl-2-thiazolyl)-phenyl-6-acetoxymethyl-1,4-dihydropyridin durch 45 Min. Kochen mit HCl in Dioxan zur 6-Hydroxymethylverbindung gespalten wird, die lactonisiert,

(c) 2-o-Formylphenyl-4-p-methoxyphenyl-thiazol, 4-Acetoxy-3-oxobuttersäureethylester und 3-Aminocrotonsäureethylester (Verfahren wie (b)),

(d) 2-Bromacetyl-o-(4-p-methoxyphenyl-2-thiazol)-zimtsäureethylester und 3-Aminocrotonsäureethylester.

Beispiel 9

Eine Lösung von 2,95 g 2-o-Formylphenyl-4-p-methoxyphenylthiazol, 2,6 g Acetessigsäureethylester und 0,1 ml Piperidin in 30 ml Isopropanol wird 10 Std. gekocht. Man kühlt ab, versetzt die erhaltene Lösung von 3,5-Diethoxycarbonyl-4-o-(4-p-methoxyphenyl-2-thiazol)-phenyl-2,6-heptandion mit 0,7 ml 33 %ig. wäßrigen Ammoniak, kocht weitere 6 Std., dampft ein, und erhält nach Chromatographie des Rückstandes an Kieselgel 2,6-Dimethyl-3,5-diethoxycarbonyl-4-o-(4-p-methoxyphenyl-2-thiazolyl)-phenyl-1,4-dihydropyridin, F. 184-186 °

Beispiel 10

Eine Lösung von 2,65 g 2-o-Formylphenyl-4-phenyl-thiazol, 1,03 g Nitroaceton und 1,15 g 3-Aminocrotonsäuremethylester in 30 ml Ethanol wird 3 Std. gekocht und eingedampft. Nach Chromatographie des Rückstandes an Kieselgel ($CH_2Cl_2/CH_3OH$ 98:2) erhält man 2,6-Dimethyl-3-methoxycarbonyl-4-o-(4-phenyl-2-thiazolyl)-phenyl-5-nitro-1,4-dihydropyridin.

Beispiel 11

Eine Lösung von 2,79 g 2-o-Formylphenyl-4-m-tolyl-thiazol, 1,29 g 3-Aminocrotonsäureethylester und 1,44 g 3-Methoxycrotonsäureethylester in 40 ml Acetonitril wird 16 Std. gekocht und eigedampft. Nach Chromatographie des Rück standes an Kieselgel erhält man 2,6-Dimethyl-3,5-diethoxycarbonyl-4-o-(4-m-tolyl-2-thiazolyl)-phenyl-1,4-dihydropyridin, F. 144-146 °.

Beispiel 12

Eine Lösung von 2,79 g 2-o-Formylphenyl-4-m-tolyl-thiazol, 1,30 g Acetessigsäureethylester, 1,44 g 3-Methoxycrotonsäureethylester und 0,7 ml 33 %ig. wäßrigem Ammoniak in 50 ml Ethanol wird 16 Std. gekocht und eingedampft. Nach Chromatographie an Kieselgel erhält man 2,6-Dimethyl-3,5-diethoxycarbonyl-4-o-(4-m-tolyl-2-thiazolyl)-phenyl-1,4-dihydropyridin, F. 144-146 °.

Beispiel 13

Eine Suspension von 3,75 g 2,6-Dimethyl-3,5-dimethoxycarbonyl-4-o-thioureidophenyl-1,4-dihydropyridin [F. 230 ° (Zers.); erhältlich durch Reaktion von 2,6-Dimethyl-3,5-dimethoxycarbonyl-4-o-aminophenyl-1,4-dihydropyridin mit Benzoylchlorid/NaSCN in Aceton bei 20 ° zur 4-o-(N'-Benzoylthioureido)-phenylverbindung (F. 203-206 ° (Zers.)) und Abspaltung der Benzoylgruppe mit $K_2CO_3$ in $CH_3OH$/Wasser bei 20 °] in 150 ml Ethanol wird mit 1,85 g 2'-Chloracetophenon 30 Min. gekocht. Nach dem Abkühlen filtriert man das erhaltene 2,6-Dimethyl-3,5-dimethoxycarbonyl-4-o-(4-phenyl-2-thiazolylamino)-phenyl-1,4-dihydropyridinhydrochlorid ab. F. 242-243 ° (Zers.).

Analog erhält man mit den entsprechenden Chlor-oder Bromcarbonylverbindungen der Formel VII die folgenden 2,6-Dimethyl-3,5-dimethoxycarbonyl-1,4-dihydropyridine:

4-o-(2-Thiazolylamino)-phenyl-, Hydrochlorid, F. 228-229 °
4-m-(2-Thiazolylamino)-phenyl-
4-o-(4-Methyl-2-thiazolylamino)-phenyl-
4-m-(4-Methyl-2-thiazolylamino)-phenyl-
4-o-(4,5-Dimethyl-2-thiazolylamino)-phenyl-, Hydrochlorid, F. 209 °
4-m-(4,5-Dimethyl-2-thiazolylamino)-phenyl-
4-o-(4-Methyl-5-phenyl-2-thiazolylamino)-phenyl-, Hydrobromid, F. 227 ° (Zers.)
4-m-(4-Methyl-5-phenyl-2-thiazolylamino)-phenyl-
4-o-(4-Ethoxycarbonyl-2-thiazolylamino)-phenyl-, Hydrobromid, F. 180-181 °
4-m-(4-Ethoxycarbonyl-2-thiazolylamino)-phenyl-
4-o-(4-Ethoxycarbonyl-5-benzyl-2-thiazolylamino)-phenyl-, F. 200-204 °
4-m-(4-Ethoxycarbonyl-5-benzyl-2-thiazolylamino)-phenyl-
4-o-(4-Ethoxycarbonylmethyl-2-thiazolylamino)-phenyl-, Hydrochlorid, F. 220-223 °
4-m-(4-Ethoxycarbonylmethyl-2-thiazolylamino)-phenyl-
4-o-[4-(2-Thienyl)-2-thiazolylamino]-phenyl-, F. 177-180 ° (Zers.)
4-o-[4-(3-Thienyl)-2-thiazolylamino]-phenyl-, F. 192-194 ° (Zers.)
4-o-[4-(2-Pyridyl)-2-thiazolylamino]-phenyl-, F. 222-224 ° (Zers.); Dihydrochlorid, F. 181-187 ° (Zers.)
4-m-[4-(2-Pyridyl)-2-thiazolylamino]-phenyl-
4-o-[4-(3-Pyridyl)-2-thiazolylamino]-phenyl-, F. 253-256 ° (Zers.); Dihydrochlorid, F. 183-192 ° (Zers.)
4-m-[4-(3-Pyridyl)-2-thiazolylamino]-phenyl-
4-o-[4-(4-Pyridyl)-2-thiazolylamino]-phenyl-, F. 248-251 ° (Zers.)
4-m-[4-(4-Pyridyl)-2-thiazolylamino]-phenyl-
4-o-[4-(3-Cyan-6-methyl-2-pyridon-5-yl)-2-thiazolylamino]-phenyl-, Hydrobromid, F. 252 °
4-m-[4-(3-Cyan-6-methyl-2-pyridon-5-yl)-2-thiazolylamino]-phenyl-
4-m-(4-Phenyl-2-thiazolylamino)-phenyl-
4-o-(4-Phenyl-5-methyl-2-thiazolylamino)-phenyl-, Hydrobromid, F. 236-237 ° (Zers.)-
4-m-(4-Phenyl-5-methyl-2-thiazolylamino)-phenyl-
4-o-(4-o-Tolyl-2-thiazolylamino)-phenyl-
4-m-(4-o-Tolyl-2-thiazolylamino)-phenyl-
4-o-(4-m-Tolyl-2-thiazolylamino)-phenyl-, Hydrobromid, F. 179-182 ° (Zers.)
4-m-(4-m-Tolyl-2-thiazolylamino)-phenyl-
4-o-(4-p-Tolyl-2-thiazolylamino)-phenyl-, Hydrobromid, F. 177-180 ° (Zers.)
4-m-(4-p-Tolyl-2-thiazolylamino)-phenyl-
4-o-[4-(2,4,6-Trimethylphenyl)-2-thiazolylamino]-phenyl-, Hydrobromid, F. 187-188 ° (Zers.)
4-m-[4-(2,4,6-Trimethylphenyl)-2-thiazolylamino]-phenyl-
4-o-[4-(2,6-Dimethyl-4-tert.-butylphenyl)-2-thiazolylamino]-phenyl-, Hydrobromid, F. 214 ° (Zers.)
4-m-[4-(2,6-Dimethyl-4-tert.-butylphenyl)-2-thiazolylamino]-phenyl-
4-o-(4-o-Methoxyphenyl-2-thiazolylamino)-phenyl-
4-m-(4-o-Methoxyphenyl-2-thiazolylamino)-phenyl-

4-o-(4-m-Methoxyphenyl-2-thiazolylamino)-phenyl-
4-m-(4-m-Methoxyphenyl-2-thiazolylamino)-phenyl-
4-o-(4-p-Methoxyphenyl-2-thiazolylamino)-phenyl-, Hydrochlorid, F. 213-214 °; Hydrobromid, F. 162-167 ° - (Zers.); Methansulfonat, F. 218-219 ° (Zers.); Sulfat, F. 209-210 ° (Zers.)
4-m-(4-p-Methoxyphenyl-2-thiazolylamino)-phenyl-
4-o-[4-(2,5-Dimethoxyphenyl)-2-thiazolylamino]-phenyl-, Hydrobromid, F. 207-210 ° (Zers.)
4-m-[4-(2,5-Dimethoxyphenyl)-2-thiazolylamino]-phenyl-
4-o-[4-(3,4-Dimethoxyphenyl)-2-thiazolylamino]-phenyl-, Hydrobromid, F. 206-208 ° (Zers.)
4-m-[4-(3,4-Dimethoxyphenyl)-2-thiazolylamino]-phenyl-
4-o-[4-(3,4-Dimethoxyphenyl)-5-methyl-2-thiazolylamino]-phenyl-, Hydrobromid, F. 218-219 ° (Zers.)
4-m-[4-(3,4-Dimethoxyphenyl)-5-methyl-2-thiazolylamino]-phenyl-
4-o-[4-(3,4,5-Trimethoxyphenyl)-2-thiazolylamino]-phenyl-, Hydrobromid, F. 210-212 ° (Zers.)
4-m-[4-(3,4,5-Trimethoxyphenyl)-2-thiazolylamino]-phenyl-
4-o-(4-p-Ethoxyphenyl-2-thiazolylamino)-phenyl-, Hydrobromid, F. 185-187 ° (Zers.)
4-m-(4-p-Ethoxyphenyl-2-thiazolylamino)-phenyl-
4-o-(4-o-Fluorphenyl-2-thiazolylamino)-phenyl-
4-m-(4-o-Fluorphenyl-2-thiazolylamino)-phenyl-
4-o-(4-m-Fluorphenyl-2-thiazolylamino)-phenyl-
4-m-(4-m-Fluorphenyl-2-thiazolylamino)-phenyl-
4-o-(4-p-Fluorphenyl-2-thiazolylamino)-phenyl-, Hydrobromid, F. 184-186 ° (Zers.)
4-m-(4-p-Fluorphenyl-2-thiazolylamino)-phenyl-
4-o-(4-o-Chlorphenyl-2-thiazolylamino)-phenyl-, Hydrobromid, F. 216-217 ° (Zers.)
4-m-(4-o-Chlorphenyl-2-thiazolylamino)-phenyl-
4-o-(4-m-Chlorphenyl-2-thiazolylamino)-phenyl-, Hydrobromid, F. 178-180 ° (Zers.)
4-m-(4-m-Chlorphenyl-2-thiazolylamino)-phenyl-
4-o-(4-p-Chlorphenyl-2-thiazolylamino)-phenyl-, Hydrobromid, F. 173-175 ° (Zers.)
4-m-(4-p-Chlorphenyl-2-thiazolylamino)-phenyl-
4-o-(4-p-Chlorphenyl-5-methyl-2-thiazolylamino)-phenyl-, Hydrobromid, F. 236-237 ° (Zers.)
4-m-(4-p-Chlorphenyl-5-methyl-2-thiazolylamino)-phenyl-
4-o-[4-(2,4-Dichlorphenyl)-2-thiazolylamino]-phenyl-, Hydrochlorid, F. 212-215 ° (Zers.)
4-m-[4-(2,4-Dichlorphenyl)-2-thiazolylamino]-phenyl-
4-o-[4-(3,4-Dichlorphenyl)-2-thiazolylamino]-phenyl-, Hydrobromid, F. 195-197 ° (Zers.)
4-m-[4-(3,4-Dichlorphenyl)-2-thiazolylamino]-phenyl-
4-o-(4-p-Bromphenyl-2-thiazolylamino)-phenyl-
4-m-(4-p-Bromphenyl-2-thiazolylamino)-phenyl-
4-o-[4-(2,4-Dimethoxy-5-bromphenyl)-2-thiazolylamino]-phenyl-, Hydrobromid, F. 245-246 ° (Zers.)
4-m-[4-(2,4-Dimethoxy-5-bromphenyl)-2-thiazolylamino]-phenyl-
4-o-(4-m-Trifluormethylphenyl-2-thiazolylamino)-phenyl-, Hydrobromid, F. 193-195 ° (Zers.)
4-m-(4-m-Trifluormethylphenyl-2-thiazolylamino)-phenyl-
4-o-(4-o-Hydroxyphenyl-2-thiazolylamino)-phenyl-
4-m-(4-o-Hydroxyphenyl-2-thiazolylamino)-phenyl-
4-o-(4-m-Hydroxyphenyl-2-thiazolylamino)-phenyl-
4-m-(4-m-Hydroxyphenyl-2-thiazolylamino)-phenyl-
4-o-(4-p-Hydroxyphenyl-2-thiazolylamino)-phenyl-, Hydrobromid, F. 219-220 ° (Zers.)
4-m-(4-p-Hydroxyphenyl-2-thiazolylamino)-phenyl-
4-o-[4-(3,4-Dihydroxyphenyl)-2-thiazolylamino]-phenyl-, Hydrochlorid, F. 234-235 ° (Zers.)
4-m-[4-(3,4-Dihydroxyphenyl)-2-thiazolylamino]-phenyl-
4-o-(4-o-Nitrophenyl-2-thiazolylamino)-phenyl-, Hydrobromid, F. 170-173 ° (Zers.)
4-m-(4-o-Nitrophenyl-2-thiazolylamino)-phenyl-
4-o-(4-m-Nitrophenyl-2-thiazolylamino)-phenyl-, Hydrobromid, F. 186-188 ° (Zers.)
4-m-(4-m-Nitrophenyl-2-thiazolylamino)-phenyl-
4-o-(4-p-Nitrophenyl-2-thiazolylamino)-phenyl-, Hydrobromid, F. 177-179 ° (Zers.)
4-m-(4-p-Nitrophenyl-2-thiazolylamino)-phenyl-
4-o-[4-(3-Nitro-4-methoxyphenyl)-2-thiazolylamino]-phenyl-, Hydrobromid, F. 195-197 ° (Zers.)
4-m-[4-(3-Nitro-4-methoxyphenyl)-2-thiazolylamino]-phenyl-
4-o-(4-p-Dimethylaminophenyl-2-thiazolylamino)-phenyl-
4-m-(4-p-Dimethylaminophenyl-2-thiazolylamino)-phenyl-
4-o-(4-p-Acetamidophenyl-2-thiazolylamino)-phenyl-

4-m-(4-p-Acetamidophenyl-2-thiazolylamino)-phenyl-
4-o-[4-(3-Acetamido-4-methoxyphenyl)-2-thiazolylamino]-phenyl-, Hydrobromid, F. 210 ° (Zers.)
4-m-[4-(3-Acetamido-4-methoxyphenyl)-2-thiazolylamino]-phenyl-
4-o-(4-p-Cyanphenyl-2-thiazolylamino)-phenyl-, Hydrobromid, F. 178-180 ° (Zers.)
4-m-(4-p-Cyanphenyl-2-thiazolylamino)-phenyl-
4-o-(4-p-Carbamoylphenyl-2-thiazolylamino)-phenyl-
4-m-(4-p-Carbamoylphenyl-2-thiazolylamino)-phenyl-
4-o-(4-o-Sulfamoylphenyl-2-thiazolylamino)-phenyl-
4-m-(4-o-Sulfamoylphenyl-2-thiazolylamino)-phenyl-
4-o-(4-m-Sulfamoylphenyl-2-thiazolylamino)-phenyl-, Hydrobromid, F. 155-157 ° (Zers.)
4-m-(4-m-Sulfamoylphenyl-2-thiazolylamino)-phenyl-
4-o-(4-p-Sulfamoylphenyl-2-thiazolylamino)-phenyl-, Hydrobromid, F. 254-256 ° (Zers.)
4-m-(4-p-Sulfamoylphenyl-2-thiazolylamino)-phenyl-
4-o-(4-p-Difluormethoxyphenyl-2-thiazolylamino)-phenyl-
4-m-(4-p-Difluormethoxyphenyl-2-thiazolylamino)-phenyl-
4-o-[4-(4-Biphenylyl)-2-thiazolylamino]-phenyl-, Hydrobromid, F. 184-189 ° (Zers.)
4-m-[4-(4-Biphenylyl)-2-thiazolylamino]-phenyl-
4-o-(4-p-Phenoxyphenyl-2-thiazolylamino)-phenyl-
4-m-(4-p-Phenoxyphenyl-2-thiazolylamino)-phenyl-.

Beispiel 14

Analog Beispiel 13 erhält man mit 2,6-Dimethyl-3,5-diethoxycarbonyl-4-o-thioureidophenyl-1,4-dihydropyridin [F. 217-218 ° (Zers.); erhältlich aus 2,6-Dimethyl-3,5-diethoxycarbonyl-4-o-aminophenyl-1,4-dihydropyridin über das 4-o-N'-Benzoylthioureidoderivat (F. 184-186 °)] bzw. mit 2,6-Dimethyl-3,5-diethoxycarbonyl-4-m-thioureidophenyl-1,4-dihydropyridin (F. 198 °) die folgenden 2,6-Dimethyl-3,5-diethoxycarbonyl-1,4-dihydropyridine:
4-o-(2-Thiazolylamino)-phenyl-
4-m-(2-Thiazolylamino)-phenyl-
4-o-(4-Methyl-2-thiazolylamino)-phenyl-, Hydrochlorid, F. 179-183 °
4-m-(4-Methyl-2-thiazolylamino)-phenyl-, Hydrochlorid, F 208-210 °
4-o-(4,5-Dimethyl-2-thiazolylamino)-phenyl-
4-m-(4,5-Dimethyl-2-thiazolylamino)-phenyl-, Hydrochlorid, F. 205-207 °
4-o-(4-Methyl-5-phenyl-2-thiazolylamino)-phenyl-, Hydrobromid, F. 219-221 ° (Zers.)
4-m-(4-Methyl-5 phenyl-2-thiazolylamino)-phenyl-
4-o-(4-Ethoxycarbonyl-2-thiazolylamino)-phenyl-
4-m-(4-Ethoxycarbonyl-2-thiazolylamino)-phenyl-, Hydrobromid, F. 137-139 °
4-o-(4-Ethoxycarbonyl-5-benzyl-2-thiazolylamino)-phenyl-, Hydrobromid, F. 212-213 ° (Zers.)
4-m-(4-Ethoxycarbonyl-5-benzyl-2-thiazolylamino)-phenyl-
4-o-(4-Ethoxycarbonylmethyl-2-thiazolylamino)-phenyl-
4-m-(4-Ethoxycarbonylmethyl-2-thiazolylamino)-phenyl-, Hydrochlorid, F. 192-193 °
4-o-(4-Carbamoyl-2-thiazolylamino)-phenyl-
4-o-(4-N-Methylcarbamoyl-2-thiazolylamino)-phenyl-
4-o-(4-N,N-Dimethylcarbamoyl-2-thiazolylamino)-phenyl-
4-o-(4-Carboxy-2-thiazolylamino)-phenyl-
4-o-(4-Benzyl-2-thiazolylamino)-phenyl-
4-o-[4-(2-Thienyl)-2-thiazolylamino]-phenyl-, F. 142-144 ° (Zers.)
4-o-[4-(3-Thienyl)-2-thiazolylamino]-phenyl-, F. 140-144 ° (Zers.)
4-o-[4-(2-Pyridyl)-2-thiazolylamino]-phenyl-, F. 165 ° (Zers.)
4-m-[4-(2-Pyridyl)-2-thiazolylamino]-phenyl-
4-o-[4-(3-Pyridyl)-2-thiazolylamino]-phenyl-, F. 170-175 ° (Zers.)
4-m-[4-(3-Pyridyl)-2-thiazolylamino]-phenyl-
4-o-[4-(4-Pyridyl)-2-thiazolylamino]-phenyl-, F. 250-252 ° (Zers.); Dihydrochlorid, F. 163-170 ° (Zers.)
4-m-[4-(4-Pyridyl)-2-thiazolylamino]-phenyl-
4-o-[4-(3-Cyan-6-methyl-2-pyridon-5-yl)-2-thiazolylamino]-phenyl-
4-m-[4-(3-Cyan-6-methyl-2-pyridon-5-yl)-2-thiazolylamino]-phenyl-, Hydrobromid, F. 120 ° (Zers.)
4-o-(4-Phenyl-2-thiazolylamino)-phenyl-, Hydrobromid, F. 243-245 ° (Zers.)

4-m-(4-Phenyl-2-thiazolylamino)-phenyl-
4-o-(4-Phenyl-5-methyl-2-thiazolylamino)-phenyl-, Hydrobromid, F. 204-205 ° (Zers.)
4-m-(4-Phenyl-5-methyl-2-thiazolylamino)-phenyl-
4-o-(4-o-Tolyl-2-thiazolylamino)-phenyl-
4-m-(4-o-Tolyl-2-thiazolylamino)-phenyl-
4-o-(4-m-Tolyl-2-thiazolylamino)-phenyl-, Hydrobromid, F. 218-220 ° (Zers.)
4-m-(4-m-Tolyl-2-thiazolylamino)-phenyl-
4-o-(4-p-Tolyl-2-thiazolylamino)-phenyl-, Hydrobromid, F. 207-209 ° (Zers.)
4-m-(4-p-Tolyl-2-thiazolylamino)-phenyl-
4-o-[4-(2,4,6-Trimethylphenyl)-2-thiazolylamino]-phenyl-, Hydrobromid, F. 218-220 ° (Zers.)
4-m-[4-(2,4,6-Trimethylphenyl)-2-thiazolylamino]-phenyl-
4-o-[4-(2,6-Dimethyl-4-tert.-butylphenyl)-2-thiazolylamino]-phenyl-, Hydrobromid, F. 243 ° (Zers.)
4-m-[4-(2,6-Dimethyl-4-tert.-butylphenyl)-2-thiazolylamino]-phenyl-
4-o-(4-o-Methoxyphenyl-2-thiazolylamino)-phenyl-
4-m-(4-o-Methoxyphenyl-2-thiazolylamino)-phenyl-
4-o-(4-m-Methoxyphenyl-2-thiazolylamino)-phenyl-
4-m-(4-m-Methoxyphenyl-2-thiazolylamino)-phenyl-
4-o-(4-p-Methoxyphenyl-2-thiazolylamino)-phenyl-, F. 213-215 ° (Zers.); Hydrobromid, F. 205-210 ° (Zers.); Hydrochlorid, F. 227-229 ° (Zers.); Methansulfonat, F. 202-203 ° (Zers.) Sulfat, F. 194-196 ° (Zers.)
4-m-(4-p-Methoxyphenyl-2-thiazolylamino)-phenyl-
4-o-[4-(2,5-Dimethoxyphenyl)-2-thiazolylamino]-phenyl-, Hydrobromid, F. 209-210 ° (Zers.)
4-m-[4-(2,5-Dimethoxyphenyl)-2-thiazolylamino]-phenyl-
4-o-[4-(3,4-Dimethoxyphenyl)-2-thiazolylamino]-phenyl-, Hydrobromid, F. 191-193 ° (Zers.)
4-m-[4-(3,4-Dimethoxyphenyl)-2-thiazolylamino]-phenyl-
4-o-[4-(3,4-Dimethoxyphenyl)-5-methyl-2-thiazolylamino]-phenyl-, Hydrobromid, F. 211-212 ° (Zers.)
4-m-[4-(3,4-Dimethoxyphenyl)-5-methyl-2-thiazolylamino]-phenyl-
4-o-[4-(3,4,5-Trimethoxyphenyl)-2-thiazolylamino]-phenyl-, Hydrobromid, F. 217-218 ° (Zers.)
4-m-[4-(3,4,5-Trimethoxyphenyl)-2-thiazolylamino]-phenyl-
4-o-(4-p-Ethoxyphenyl-2-thiazolylamino)-phenyl-, Hydrobromid, F. 208-210 ° (Zers.)
4-m-(4-p-Ethoxyphenyl-2-thiazolylamino)-phenyl-
4-o-(4-p-Methylthiophenyl-2-thiazolylamino)-phenyl-
4-o-(4-p-Methylsulfinylphenyl-2-thiazolylamino)-phenyl-
4-o-(4-p-Methylsulfonylphenyl-2-thiazolylamino)-phenyl-
4-o-(4-o-Fluorphenyl-2-thiazolylamino)-phenyl-
4-m-(4-o-Fluorphenyl-2-thiazolylamino)-phenyl-
4-o-(4-m-Fluorphenyl-2-thiazolylamino)-phenyl-
4-m-(4-m-Fluorphenyl-2-thiazolylamino)-phenyl-
4-o-(4-p-Fluorphenyl-2-thiazolylamino)-phenyl-, Hydrobromid, F. 242-244 ° (Zers.)
4-m-(4-p-Fluorphenyl-2-thiazolylamino)-phenyl-
4-o-(4-o-Chlorphenyl-2-thiazolylamino)-phenyl-, Hydrobromid, F. 217-218 ° (Zers.)
4-m-(4-o-Chlorphenyl-2-thiazolylamino)-phenyl-
4-o-(4-m-Chlorphenyl-2-thiazolylamino)-phenyl-, Hydrobromid, F. 204-206 ° (Zers.)
4-m-(4-m-Chlorphenyl-2-thiazolylamino)-phenyl-
4-o-(4-p-Chlorphenyl-2-thiazolylamino)-phenyl-, Hydrobromid, F. 200-203 ° (Zers.)
4-m-(4-p-Chlorphenyl-2-thiazolylamino)-phenyl-
4-o-(4-p-Chlorphenyl-5-methyl-2-thiazolylamino)-phenyl-, Hydrobromid, F. 200-201 ° (Zers.)
4-m-(4-p-Chlorphenyl-5-methyl-2-thiazolylamino)-phenyl-
4-o-[4-(2,4-Dichlorphenyl)-2-thiazolylamino]-phenyl-, Hydrochlorid, F. 220-223 ° (Zers.)
4-m-[4-(2,4-Dichlorphenyl)-2-thiazolylamino]-phenyl-
4-o-[4-(3,4-Dichlorphenyl)-2-thiazolylamino]-phenyl-, Hydrobromid, F. 205-209 ° (Zers.)
4-m-[4-(3,4-Dichlorphenyl)-2-thiazolylamino]-phenyl-
4-o-(4-p-Bromphenyl-2-thiazolylamino)-phenyl-
4-m-(4-p-Bromphenyl-2-thiazolylamino)-phenyl-
4-o-[4-(2,4-Dimethoxy-5-bromphenyl)-2-thiazolylamino]-phenyl-, Hydrobromid, F. 216-218 ° (Zers.)
4-m-[4-(2,4-Dimethoxy-5-bromphenyl)-2-thiazolylamino]-phenyl-
4-o-(4-p-Jodphenyl-2-thiazolylamino)-phenyl-
4-o-(4-m-Trifluormethylphenyl-2-thiazolylamino)-phenyl-, Hydrobromid, F. 205 ° (Zers.)
4-m-(4-m-Trifluormethylphenyl-2-thiazolylamino)-phenyl-

4-o-(4-o-Hydroxyphenyl-2-thiazolylamino)-phenyl-
4-m-(4-o-Hydroxyphenyl-2-thiazolylamino)-phenyl-
4-o-(4-m-Hydroxyphenyl-2-thiazolylamino)-phenyl-
4-m-(4-m-Hydroxyphenyl-2-thiazolylamino)-phenyl-
4-o-(4-p-Hydroxyphenyl-2-thiazolylamino)-phenyl-, Hydrobromid, F. 193-194 ° (Zers.)
4-m-(4-p-Hydroxyphenyl-2-thiazolylamino)-phenyl-
4-o-[4-(3,4-Dihydroxyphenyl)-2-thiazolylamino]-phenyl-, Hydrochlorid, F. 198-202 ° (Zers.)
4-m-[4-(3,4-Dihydroxyphenyl)-2-thiazolylamino]-phenyl-
4-o-(4-o-Nitrophenyl-2-thiazolylamino)-phenyl-, Hydrobromid, F. 188-190 ° (Zers.)
4-m-(4-o-Nitrophenyl-2-thiazolylamino)-phenyl-
4-o-(4-m-Nitrophenyl-2-thiazolylamino)-phenyl-, Hydrobromid, F. 190-191 ° (Zers.)
4-m-(4-m-Nitrophenyl-2-thiazolylamino)-phenyl-
4-o-(4-p-Nitrophenyl-2-thiazolylamino)-phenyl-, Hydrobromid, F. 191-192 ° (Zers.)
4-m-(4-p-Nitrophenyl-2-thiazolylamino)-phenyl-
4-o-[4-(3-Nitro-4-methoxyphenyl)-2-thiazolylamino]-phenyl-, Hydrobromid, F. 215-217 °
4-m-[4-(3-Nitro-4-methoxyphenyl)-2-thiazolylamino]-phenyl-
4-o-(4-p-Dimethylaminophenyl-2-thiazolylamino)-phenyl-
4-m-(4-p-Dimethylaminophenyl-2-thiazolylamino)-phenyl-
4-o-(4-p-Acetamidophenyl-2-thiazolylamino)-phenyl-
4-m-(4-p-Acetamidophenyl-2-thiazolylamino)-phenyl-
4-o-[4-(3-Acetamido-4-methoxyphenyl)-2-thiazolylamino]-phenyl-, Hydrobromid, F. 230 ° (Zers.)
4-m-[4-(3-Acetamido-4-methoxyphenyl)-2-thiazolylamino]-phenyl-
4-o-(4-p-Cyanphenyl-2-thiazolylamino)-phenyl-, Hydrobromid, F. 198-200 ° (Zers.)
4-m-(4-p-Cyanphenyl-2-thiazolylamino)-phenyl-
4-o-(4-p-Carbamoylphenyl-2-thiazolylamino)-phenyl-
4-m-(4-p-Carbamoylphenyl-2-thiazolylamino)-phenyl-
4-o-(4-o-Sulfamoylphenyl-2-thiazolylamino)-phenyl-
4-m-(4-o-Sulfamoylphenyl-2-thiazolylamino)-phenyl-
4-o-(4-m-Sulfamoylphenyl-2-thiazolylamino)-phenyl-, Hydrobromid, F. 211-212 ° (Zers.)
4-m-(4-m-Sulfamoylphenyl-2-thiazolylamino)-phenyl-
4-o-(4-p-Sulfamoylphenyl-2-thiazolylamino)-phenyl-, Hydrobromid, F. 248-250 ° (Zers.)
4-m-(4-p-Sulfamoylphenyl-2-thiazolylamino)-phenyl-
4-o-(4-p-Difluormethoxyphenyl-2-thiazolylamino)-phenyl-
4-m-(4-p-Difluormethoxyphenyl-2-thiazolylamino)-phenyl-
4-o-[4-(4-Biphenylyl)-2-thiazolylamino]-phenyl-, Hydrobromid, F. 206-209 ° (Zers.)
4-m-[4-(4-Biphenylyl)-2-thiazolylamino]-phenyl-
4-o-(4-p-Phenoxyphenyl-2-thiazolylamino)-phenyl-
4-m-(4-p-Phenoxyphenyl-2-thiazolylamino)-phenyl-.

Beispiel 15

Ein Gemisch von 4,3 g 2,6-Dimethyl-3,5-dimethoxycarbonyl-4-o-(N'-dimethylaminomethylen-thioureido)-phenyl-1,4-dihydropyridin [F. 208-209 ° (Zers.); erhältlich durch 1,5 Std. Kochen von 2,6-Dimethyl-3,5-dimethoxycarbonyl-4-o-thioureidophenyl-1,4-dihydropyridin und N,N-Dimethylformamiddimethylacetal in Acetonitril], 1,26 g Benzylchlorid, 0,6 g NaH (80 %ig) und 30 ml DMF wird 3 Std. bei 20 ° gerührt. Nach dem Eindampfen wird Wasser zugegeben und das erhaltene 2,6-Dimethyl-3,5-dimethoxycarbonyl-4-o-(5-phenyl-2-thiazolylamino)-phenyl-1,4-dihydropyridin abfiltriert und mit Wasser gewaschen.

Beispiel 16

Ein Gemisch von 572 mg 2,6-Dimethyl-3,5-dimethoxycarbonyl-4-o-(4-p-hydroxyphenyl-2-thiazolylamino)-phenyl-1,4-dihydropyridin-hydrobromid, 140 mg 2-Dimethylaminoethylchlorid-hydrochlorid, 560 mg $K_2CO_3$, 40 mg Tetrabutylammoniumjodid und 20 ml Acetonitril wird 24 Std. bei 20 ° gerührt. Man saugt das ausgefallene 2,6-Dimethyl-3,5-dimethoxycarbonyl-4-o-[4-p-(2-dimethylaminoethoxy)-phenyl-2-thiazolylamino]-phenyl-1,4-dihydropyridin ab und wäscht es mit Wasser.

Beispiel 17

Eine Lösung von 4,88 g 2,6-Dimethyl-3,5-diethoxycarbonyl-4-o-(4-phenyl-2-thiazolyl)-phenyl-1,4-dihydropyridin in 45 ml DMF wird mit 0,45 g 80 %igem NaH versetzt. Nach halbstündigem Rühren gibt man 1,5 g 1-(2-Chlorethyl)-morpholin in 10 ml DMF hinzu und rührt noch 4 Std. bei 20°. Nach üblicher Aufarbeitung (Ethylacetat) erhält man 1-(2-Morpholinoethyl)-2,6-dimethyl-3,5-diethoxycarbonyl-4-o-(4-phenyl-2-thiazolyl)-phenyl-1,4-dihydropyridin.

Beispiel 18

Eine Lösung von 0,5 g 2,6-Dimethyl-3,5-diethoxycarbonyl-4-o-(4-p-nitrophenyl-2-thiazolyl)-phenyl-1,4-dihydropyridin in 50 ml Methanol wird an 0,5 g Raney-Nickel bei 1 bar und 20° bis zum Stillstand hydriert. Man filtriert, dampft ein und erhält 2,6-Dimethyl-3,5-diethoxycarbonyl-4-o-(4-p-aminophenyl-2-thiazolyl-phenyl-1,4-dihydropyridin.

Aus den entsprechenden Nitroderivaten erhält man analog die nachstehenden 2,6-Dimethyl-3,5-diethoxycarbonyl-1,4-dihydropyridine:
4-o-(4-o-Aminophenyl-2-thiazolyl)-phenyl-
4-m-(4-o-Aminophenyl-2-thiazolyl)-phenyl-
4-o-(4-m-Aminophenyl-2-thiazolyl)-phenyl-
4-m-(4-m-Aminophenyl-2-thiazolyl)-phenyl-
4-m-(4-p-Aminophenyl-2-thiazolyl)-phenyl-.

Beispiel 19

a) Eine Lösung von 5,03 g 2,6-Dimethyl-3,5-diethoxycarbonyl-4-o-(4-phenyl-2-thiazolylamino)-phenyl-1,4-dihydropyridin in 150 ml Dichlormethan wird bei 0-5° mit 1,4 ml Pyridin und 3,84 g Pyridiniumbromidperbromid versetzt. Man rührt 3 Std. bei 4°, arbeitet wie üblich auf und erhält 2-Brommethyl-3,5-diethoxycarbonyl-4-o-(4-phenyl-2-thiazolylamino)-phenyl-6-methyl-1,4-dihydropyridin.

b) Eine Lösung von 0,56 g der nach a) erhaltenen Bromverbindung in 30 ml Acetonitril wird 45 Min. gekocht. Nach dem Eindampfen und chromatographischer Reinigung erhält man 2-Methyl-3-ethoxycarbonyl-4-o-(4-phenyl-2-thiazolylamino)-phenyl-5-oxo-1,4,5,7-tetrahydrofuro[3,4-b]pyridin und 2-Hydroxymethyl-3,5-diethoxycarbonyl-4-o-(4-phenyl-2-thiazolylamino)-phenyl-6-methyl-1,4-dihydropyridin.

Beispiel 20

Eine Lösung von 6,49 g 2-(2-Phthalimidoethoxymethyl)-3,5-dimethoxycarbonyl-4-o-(4-phenyl-2-thiazolyl)-phenyl-6-methyl-1,4-dihydropyridin [erhältlich aus 3-Oxo-4-(2-phthalimidoethoxy)-butansäuremethylester, 2-(o-Formylphenyl)-4-phenylthiazol und 3-Aminocrotonsäuremethylester] und 0,6 g Hydrazinhydrat in 80 ml Ethanol wird 3 Std. bei 20° gerührt und eingeengt. Das ausgefallene 2-(2-o-Hydrazinocarbonylbenzamido-ethoxymethyl)-3,5-dimethoxycarbonyl-4-o-(4-phenyl-2-thiazolyl)-phenyl-6-methyl-1,4-dihydropyridin wird anschließend mit 100 ml methanolischem HCl 1 Std. gekocht. Man dampft ein, löst in 50 %igem Ethanol, filtriert, macht alkalisch bis pH 9 und arbeitet wie üblich auf. Man erhält 2-(2-Aminoethoxymethyl)-3,5-dimethoxycarbonyl-4-o-(4-phenyl-2-thiazolyl)-phenyl-6-methyl-1,4-dihydropyridin.

Beispiel 21

Analog Beispiel 13 erhält man aus 2-Methyl-3-methoxycarbonyl-4-o-thioureidophenyl-5-oxo-1,4,5,7-tetrahydrofuro-[3,4-b]pyridin [F. 217-219 °; erhältlich durch Hydrierung von 2-Methyl-3-methoxycarbonyl-4-o-nitrophenyl-5-oxo-1,4,5,7-tetrahydrofuro[3,4-b]pyridin an Pd-C in DMF zur 4-o-Aminophenylverbindung (F. 183-184 °), Reaktion mit Benzoyliso thiocyanat in Aceton bei 20 ° zur 4-o-(N'-Benzoylthioureido)-phenylverbindung (F. 170-174°) und Abspaltung der Benzoylgruppe] und 2'-Chloracetophenon das 2-Methyl-3-methoxycarbonyl-4-o-(4-phenyl-2-thiazolylamino)-phenyl-5-oxo-1,4,5,7-tetrahydofuro[3,4-b]pyridin, Hydrochlorid, F. 221 ° (Zers.)

Analog erhält man die folgenden 2-Methyl-3-methoxycarbonyl-5-oxo-1,4,5,7-tetrahydofuro[3,4-b]-pyridine:
4-o-(4-Methyl-2-thiazolylamino)-phenyl-, Hydrochlorid, F. 180-181 ° (Zers.)
4-o-(4-Ethoxycarbonyl-2-thiazolylamino)-phenyl-, Hydrobromid, F. 230-231 ° (Zers.)
4-o-[4-(2-Thienyl)-2-thiazolylamino]-phenyl-, Hydrobromid, F. 219-220 ° (Zers.)
4-o-[4-(3-Thienyl)-2-thiazolylamino]-phenyl-, Hydrobromid, F. 238-240 ° (Zers.)
4-o-(4-m-Tolyl-2-thiazolylamino)-phenyl-, Hydrobromid, F. 218-219 ° (Zers.)
4-o-(4-p-Tolyl-2-thiazolylamino)-phenyl-, Hydrobromid, F. 237-239 ° (Zers.)
4-o-(4-m-Methoxyphenyl-2-thiazolylamino)-phenyl-, Hydrobromid, F. 218-219 ° (Zers.)
4-o-(4-p-Methoxyphenyl-2-thiazolylamino)-phenyl-, Hydrochlorid, F. 233-234 ° (Zers.)
4-o-(4-p-Fluorphenyl-2-thiazolylamino)-phenyl-, Hydrobromid, F. 227-228 ° (Zers.)
4-o-(4-o-Chlorphenyl-2-thiazolylamino)-phenyl-, Hydrobromid, F. 223-224 ° (Zers.)
4-o-(4-m-Chlorphenyl-2-thiazolylamino)-phenyl-, Hydrobromid, F. 209-211 ° (Zers.)
4-o-(4-p-Chlorphenyl-2-thiazolylamino)-phenyl-, Hydrobromid, F. 227-229 ° (Zers.)

Beispiel 22

Analog Beispiel 13 erhält man aus 1,7-Dioxo-8-o-thioureidophenyl-1,3,4,5,7,8-hexahydro-difuro[3,4-b:3'4'-e]pyridin [F. 229-230 ° (Zers.); erhältlich durch Bromierung von 2-Methyl-3-methoxycarbonyl-4-o-nitrophenyl-5-oxo-1,4,5,7-tetrahydro-furo[3,4-b]pyridin mit Pyridinium-hydrobromidperbromid/Triethylamin in THF zur 2-Brommethyl-verbindung, Cyclisierung in Acetonitril bei 82 ° zu 1,7-Dioxo-8-o-nitrophenyl-1,3,4,5,7,8-hexahydro-difuro[3,4-b:3',4'-e]-pyridin (F. 273-274 °), Hydrierung zur 8-o-Aminophenylverbindung (F. 285-287 °, Zers.), Reaktion mit Benzoylisothiocyanat zur 8-o-(N'-Benzoylthioureido)-phenylverbindung (F. 219-220 °, Zers.) und Abspaltung der Benzoylgruppe] das 1,7-Dioxo-8-o-(4-phenyl-2-thiazolylamino)-phenyl-1,3,4,5,7,8-hexahydro-difuro[3,4-b:3',4'-e]pyridin, Hydrochlorid, F. 264-268 ° (Zers.)

Analog erhält man die folgenden 1,7-Dioxo-1,3,4,5,7,8-hexahydro-difuro[3,4-b:3',4'-e]pyridine:
8-o-[4-(2-Thienyl)-2-thiazolylamino]-phenyl-, Hydrobromid, F. 263-265 ° (Zers.)
8-o-[4-(3-Thienyl)-2-thiazolylamino]-phenyl-, Hydrobromid, F. 295-297 ° (Zers.)
8-o-(4-m-Tolyl-2-thiazolylamino)-phenyl-, F. 220 ° (Zers.)
8-o-(4-p-Tolyl-2-thiazolylamino)-phenyl-, Hydrobromid, F. 226-229 ° (Zers.)
8-o-(4-m-Methoxyphenyl-2-thiazolylamino)-phenyl-, Hydrobromid, F. 236-239 ° (Zers.)
8-o-(4-p-Methoxyphenyl-2-thiazolylamino)-phenyl-, Hydrochlorid, F. 239 ° (Zers.)
8-o-(4-p-Fluorphenyl-2-thiazolylamino)-phenyl-, Hydrobromid, F. 274-275 ° (Zers.)
8-o-(4-o-Chlorphenyl-2-thiazolylamino)-phenyl-, Hydrobromid, F. 277 ° (Zers.)
8-o-(4-m-Chlorphenyl-2-thiazolylamino)-phenyl-, Hydrobromid, F. 268-270 ° (Zers.)
8-o-(4-p-Chlorphenyl-2-thiazolylamino)-phenyl-, Hydrobromid, F. 285-286 ° (Zers.)

Beispiel 23

Ein Gemisch von 0,44 g 1,7-Dioxo-8-o-(4-phenyl-2-thiazolylamino)-phenyl-1,3,4,5,7,8-hexahydro-difuro-[3,4-b:3',4'-e]-pyridin, 0,25 ml Benzylbromid, 0,69 g $K_2CO_3$, 2 ml DMF und ml THF wird bei 20 ° 8 Std. gerührt. Nach Filtration, Eindampfen und Chromatographie des harzigen Rückstands an Kieselgel mit Ethylacetat erhält man 4-Benzyl-1,7-dioxo-8-o-(4-phenyl-2-thiazolylamino)-phenyl-1,3,4,5,7,8-hexahydro-difuro[3,4-b:3',4'-e]pyridin, F. 162-165 ° (Zers.)

Die nachstehenden Beispiele betreffen pharmazeutische Zubereitungen, die Verbindungen der Formel I oder ihre physiologisch unbedenklichen Salze enthalten:

Beispiel A: Tabletten

Ein Gemisch von 1 kg 2,6-Dimethyl-3,5-diethoxycarbonyl-4-o-(4-p-chlorphenyl-2-thiazolylamino)-phenyl-1,4-dihydropyridin-hydrobromid, 4 kg Lactose, 1,2 kg Kartoffelstärke, 0,2 kg Talk und 0,1 kg Magnesiumstearat wird in üblicher Weise zu Tabletten gepreßt, derart, daß jede Tablette 5 mg Wirkstoff enthält.

Beispiel B: Dragees

Analog Beispiel A werden Tabletten gepreßt, die anschließend in üblicher Weise mit einem Überzug aus Saccharose, Kartoffelstärke, Talk, Tragant und Farbstoff überzogen werden.

Beispiel C: Kapseln

Man füllt 10 kg 2,6-Dimethyl-3,5-dimethoxycarbonyl-4-o-(4-phenyl-2-thiazolylamino)-phenyl-1,4-dihydropyridinhydrochlorid in üblicher Weise in Hartgelatinekapseln, so daß jede Kapsel 50 mg Wirkstoff enthält.

Beispiel D: Ampullen

Ein Gemisch von 0,1 kg 2,6-Dimethyl-3,5-dimethoxycarbonyl-4-o-(4-p-fluorphenyl-2-thiazolylamino)-phenyl-1,4-dihydropyridin-hydrobromid, 0,27 kg Natriumchlorid und 30 l Polyethylenglykol 400 wird mit Wasser auf 100 l aufgefüllt, steril filtriert, in Ampullen abgefüllt und steril verschlossen. Jede Ampulle enthält 2 mg Wirkstoff.

Analog sind Tabletten, Dragees, Kapseln oder Ampullen erhältlich, die einen oder mehrere der übrigen Wirkstoffe der Formel I und/oder ihrer physiologisch unbedenklichen Salze enthalten.

**Ansprüche**

1. Dihydropyridine der Formel I

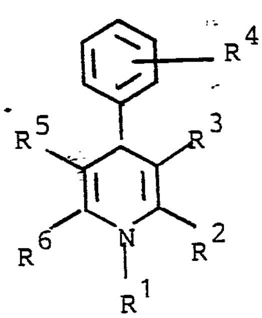

I

worin

$R^1$ H, A, Ar-alkyl, AO-alkyl, ArO-alkyl, Ar-alkyl-O-alkyl oder $R^7R^8N$-alkyl,

$R^2$ und $R^6$ jeweils H, A, Ar-alkyl, Hal-alkyl, $CF_3$, $R^9OCH_2$-, $R^7R^8N-(CH_2)_a-CHR^{10}$-, $R^{11}SO_m-CH_2$-, CN oder eine freie oder funktionell abgewandelte CHO-Gruppe,

$R^3$ und $R^5$ jeweils $R^{12}CO$-, $R^{11}SO_2$-, $(AO)_2PO$-, $NO_2$ oder CN,

$R^4$ 4-$R^{15}$-5-$R^{16}$-2-thiazolyl oder 4-$R^{15}$-5-$R^{16}$-2-thiazolylamino,
$R^7$ H, A, Ar, AOOC-, Ar-alkyl-OOC-, $R^{10}$NHCO-, $R^{11}SO_2$ oder Ac,

$R^8$ H, A oder Ar-alkyl,

$R^7$ und $R^8$ zusammen auch Z, $-COCH_2CH_2CO-$, $-COCH=CHCO-$oder $-CO-(o-C_6H_4)-CO-$,

$R^9$ H, A, Ar, Ar-alkyl, AO-alkyl, $R^7R^8$N-alkyl, Ac, $R^{10}$NHCO-, $R^{11}SO_2$-oder $CF_3SO_2$-,

$R^{10}$ H A oder Ar,

$R^7$ und $R^{10}$ zusammen auch Alkylen mit 2-4 C-Atomen,

$R^{11}$ A oder Ar,

$R^{12}$ HO, AO, $R^{14}$-alkyl-O-, Z=CH-O-, A, Ar, Het, $R^7R^8$N-oder $R^{15}$O-alkyl-NH-,

$R^9$ und $R^{12}$ sowie $R^8$ und $R^{12}$ jeweils zusammen auch eine Bindung,

$R^{13}$ und $R^{16}$ jeweils H, A, AOOC, $AOOCCH_2$, $H_2NCO$, ANHCO, $A_2NCO$, HOOC, Ar-alkyl, Het oder eine Phenylgruppe, die ein-bis dreifach substituiert sein kann durch A, AO, $ASO_m$-, Hal, $CF_3$, HO, $O_2N$, $R^7R^8N$, CN, $H_2NCO$, $H_2NSO_2$, $CHF_2$-O-, $R^7R^8$N-alkyl-O-, Ar oder ArO,

$R^{14}$ AO, ArO, Ar-alkyl-O-, $R^7R^8$N-, $R^{11}SO_2$O-oder $(AO)_2$PO-O-,

$R^{15}$ H, A, Ar oder Ac,

a und m jeweils 0, 1 oder 2,

A einen geradkettigen oder verzweigten, gesättigten oder ungesättigten aliphatischen oder cycloaliphatischen Kohlenwasserstoffrest mit 1-20 C-Atomen,

Ac $R^{10}$CO-, Ar-alkyl-CO-oder Ar-alkenyl-CO-,

Ar Phenyl; ein-bis dreifach durch A, AO, AcO, Hal, $CF_3$, HO, $O_2N$, $H_2N$, ANH, $A_2N$, AcNH, AOOCNH-, Ar-alkyl-OOCNH-, CN, $H_2NCO$, HOOC, AOOC, $H_2NSO_2$ und/oder $R^{11}$NHCONH-substituiertes Phenyl; oder Naphthyl,

Hal F, Cl, Br oder J,

Het einen 5-oder 6-gliedrigen ein-oder mehrkernigen heterocyclischen Rest mit 1-4 O-, N-und/oder S-Atomen, der ein-oder mehrfach durch A, AO, Hal, $CF_3$, HO, $O_2N$, $H_2N$, NHA, $NA_2$, AcNH, $ASO_m$, AOOC, CN, $H_2NCO$, HOOC, $H_2NSO_2$, $ASO_2NH$, Ar, Ar-alkenyl und/oder Pyridyl substituiert sein kann, oder 1-$R^1$-2-$R^2$-3-$R^3$-5-$R^5$-6-$R^6$-1,4-dihydro-4-pyridyl,

Z eine Alkylenkette mit 4 oder 5 C-Atomen, die durch O, HN, AN, ArN, Ar-alkyl-N, $Ar_2$CHN oder AcN unterbrochen sein kann und

-alkyl-bzw. -alkenyl-Alkylen-bzw. Alkenylen ketten mit jeweils 1-4 C-Atomen bedeuten,

sowie deren Salze.

2.a) 2,6-Dimethyl-3,5-dimethoxycarbonyl-4-o-(4-phenyl-2-thiazolylamino)-phenyl-1,4-dihydropyridin;

b) 2,6-Dimethyl-3,5-diethoxycarbonyl-4-o-(4-p-chlorphenyl-2-thiazolylamino)-phenyl-1,4-dihydropyridin;

c) 2,6-Dimethyl-3,5-dimethoxycarbonyl-4-o-[4-(3,4-dichlorphenyl)-2-thiazolylamino]-phenyl-1,4-dihydropyridin;

d) 2,6-Dimethyl-3,5-dimethoxycarbonyl-4-o-(4-p-fluorphenyl-2-thiazolylamino)-phenyl-1,4-dihydropyridin;

e) 2,6-Dimethyl-3,5-dimethoxycarbonyl-4-o-(4-p-methoxyphenyl-2-thiazolylamino)-phenyl-1,4-dihydropyridin;

f) 2,6-Dimethyl-3,5-dimethoxycarbonyl-4-o-(4-m-trifluormethylphenyl-2-thiazolylamino)-phenyl-1,4-dihydropyridin;

g) 2,6-Dimethyl-3,5-diethoxycarbonyl-4-o-(4-m-chlorphenyl-2-thiazolylamino)-phenyl-1,4-dihydropyridin;

h) 2,6-Dimethyl-3,5-dimethoxycarbonyl-4-o-(4-p-tolyl-2-thiazolylamino)-phenyl-1,4-dihydropyridin.

3. Verfahren zur Herstellung von Dihydropyridinen der Formel I sowie von ihren Salzen, dadurch gekennzeichnet, daß man Verbindungen der Formeln II, III, IV und V

$R^1NH_2$ II

$R^2\text{-CO-CH}_2\text{-}R^3$ III

$R^6\text{-CO-CH}_2\text{-}R^5$ IV

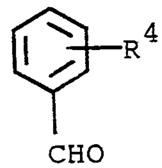

V

oder deren funktionelle Derivate, falls erwünscht stufenweise, miteinander umsetzt,

oder daß man ein Thioamid der Formel VI

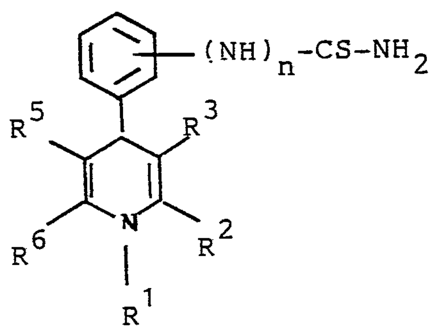

VI

worin

n 0 oder 1 beduetet,

mit einer Carbonylverbindung der Formel VII

$R^{16}\text{-CHX-CO-}R^{13}$ VII

worin

X Cl, Br, J oder eine freie oder reaktionsfähig veresterte OH-Gruppe bedeutet

umsetzt,
oder daß man eine Verbindung der Formel VIII

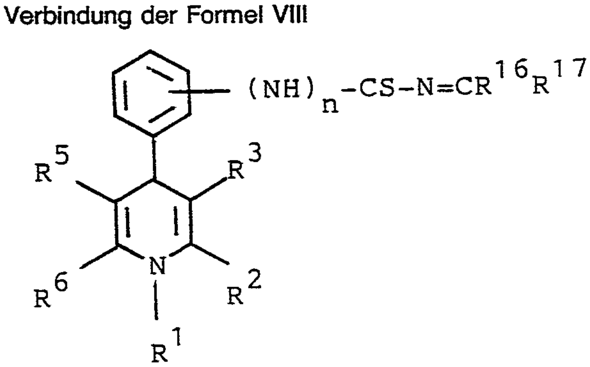

VIII

worin

$R^{17}$ HO, AO oder $A_2N$ bedeutet

mit einer Verbindung der Formel IX

$R^{13}$-$CH_2$-X IX

umsetzt,

und/oder daß man in einer Verbindung der Formel I einen oder mehrere der Reste $R^1$, $R^2$, $R^3$, $R^4$, $R^5$ und/oder $R^6$ in andere Reste $R^1$, $R^2$, $R^3$, $R^4$, $R^5$ und/oder $R^6$ umwandelt und/oder eine basische bzw. saure Verbindung der Formel I durch Behandeln mit einer Säure bzw. Base in eines ihrer Salze überführt.

4. Verfahren zu Herstellung pharmazeutischer Zubereitungen, dadurch gekennzeichnet, daß man eine Verbindung der Formel I und/oder eines ihrer physiologisch unbedenklichen Salze zusammen mit mindestens einem festen, flüssigen oder halbflüssigen Träger-oder Hilfsstoff und gegebenenfalls in Kombination mit einem oder mehreren weiteren Wirkstoff(en) in eine pharmazeutische Dosierungsform überführt.

5. Pharmazeutische Zubereitung, gekennzeichnet durch einen Gehalt an mindestens einer Verbindung der Formel I und/oder einem ihrer physiologisch unbedenklichen Salze.

6. Verbindungen der Formel I und deren physiologisch unbedenkliche Salze zur Bekämpfung von Krankheiten.

7. Verwendung von Verbindungen der Formel I oder von deren physiologisch unbedenklichen Salzen bei der Bekämpfung von Krankheiten.

8. Verwendung von Verbindungen der Formel I oder von deren physiologisch unbedenklichen Salzen zur Herstellung eines Arzneimittels.

9. Verbindungen der Formel VI.